# EUROPÄISCHE PATENTSCHRIFT

(11) **EP 1 284 716 B1**
(45) Veröffentlichungstag und Bekanntmachung des Hinweises auf die Patenterteilung: **02.11.2006**
(21) Anmeldenummer: 01957809.5
(22) Anmeldetag: 29.05.2001
(51) Int. Cl.: A61K 9/107, A61K 9/16

(54) **SELBSTEMULGIERENDE WIRKSTOFFORMULIERUNG UND VERWENDUNG DIESER FORMULIERUNG**
SELF-EMULSIFYING ACTIVE SUBSTANCE FORMULATION AND USE OF THIS FORMULATION
FORMULATION AUTOEMULSIFIANTE DE PRINCIPE ACTIF ET UTILISATION DE LADITE FORMULATION

(30) Priorität: 30.05.2000 DE 10026698
(43) Veröffentlichungstag der Anmeldung: 26.02.2003
(73) Patentinhaber: BASF Aktiengesellschaft, 67056 Ludwigshafen (DE)
(72) Erfinder: BERNDL, Gunther, 67273 Herxheim (DE); BREITENBACH, Jörg, 68199 Mannheim (DE); HEGER, Robert, 69124 Heidelberg (DE); STADLER, Michael, 68809 Neulussheim (DE); WILKE, Peter, 67063 Ludwigshafen (DE); ROSENBERG, Jörg, 67158 Ellerstadt (DE)
(74) Vertreter: Kinzebach, Werner
(86) Internationale Anmeldenummer: PCT/EP2001/006116
(87) Internationale Veröffentlichungsnummer: WO 2001/091727

(56) Entgegenhaltungen:
- EP-A- 0 570 327
- WO-A-00/00179
- DE-A- 19 531 277
- DE-A- 19 536 387
- DE-C- 973 095

## Beschreibung

Die vorliegende Erfindung betrifft selbstemulgierende Formulierungen auf Basis einer Wirkstoffkomponente und einer Formulierungsgrundlage mit einer Lipidkomponente und einer Bindemittelkomponente; die Verwendung dieser Formulierung als Dosierungsform im Life-Science-Bereich; die Erfindung beschreibt auch ein Verfahren zur Herstellung selbstemulgierender Formulierungen durch Vermischen der Formulierungskomponenten unter Bildung eines plastischen Gemisches und gegebenenfalls unter Herrichtung der Formulierungen als Dosierungsform; und die Verwendung einer Formulierungsgrundlage bei der Anwendung wenigstens eines Wirkstoffs im Life-Science-Bereich.

Vielfach ist es erwünscht, Wirkstoffe in emulgierter Form einsetzen zu können. So werden im Bereich der Galenik schwerlösliche Wirkstoffe zusammen mit ausgewählten Hilfsstoffen formuliert, um eine ausreichende Resorption des Wirkstoffs beispielsweise im Gastrointestinaltrakt zu gewährleisten. Es handelt sich dabei üblicherweise um nichtionogene Tenside mit recht hohen HLB-Werten, z.B. Cremophor®, Tween®, etc. Dies.gilt für den Pharmabereich genauso wie für den Pflanzenschutzsektor.

Beispielsweise werden in der WO 00/00.179 Öle oder Fette unter Zusatz üblicher Emulgatoren emulgiert oder mikroemulgiert und wenig wasserlösliche Wirkstoffe werden dann in diese Emulsionen bzw. Mikroemulsionen eingearbeitet.

Obwohl diese Hilfsstoffe gemeinhin als chemisch indifferent bezeichnet werden, besitzen sie bekanntermaßen Nachteile, die sich insbesondere bei höheren Dosierungen durch lokale und/oder systemische Toxizität bemerkbar machen können.

Neben lokalen Reizungen können aufgrund der Aufnahme dieser Solubilisatoren durch einen Organismus unerwünschte Nebenwirkungen dieser Substanzen nicht ausgeschlossen werden.

Emulsionen beispielsweise zur parenteralen Verabreichung verwenden in der Regel emulgierende Phospholipide, insbesondere Lecithine. Aufgrund der unzureichenden chemischen Beständigkeit der Phospholipide können diese Emulsionen allerdings erhebliche Lagerstabilitätsprobleme bereiten. Zudem ist die Herstellung solcher Emulsionen aufwendig. So kann es erforderlich sein, die Phospholipide in Wasser zusammen mit weiteren Emulsionsbestandteilen, beispielsweise Lipiden oder Lipidderivaten unter Hochdruck, d.h. bei mehreren 100 bar, zu homogenisieren.

Neben den zuvor beschriebenen flüssigen Emulsionen sind auch "feste" Emulsionen bekannt. Diese Formulierungen werden im Allgemeinen als selbstemulgierende Systeme bezeichnet, da sie sich in wässrigen Systemen unter Bildung einer Emulsion auflösen (vgl. M.O. Bachynsky et al., "Factors Affecting the Efficiency of a Self-Emulsifying Oral Delivery System", Drug Development and Industrial Pharmacy, 23 (8), (1997) 809-816; US 5,858,401). Auch hier kommen überwiegend die eingangs diskutierten solubilisationsfördernden Hilfsstoffe zur Anwendung, was die bekannten Nachteile mit sich bringt. Neben den vor allen zur Anwendung kommenden niedermolekularen Tensiden, z.B. Tween®, werden auch selbstemulgierende Systeme auf Basis polymerer Glycerid-Tenside beschrieben (A.T.M. Serajuddin, "Bioavailability Enhancement of poorly Water-Soluble Drugs by Solid Dispersion in Surface Active and Self-Emulsifying Vehicles", Bulletin Technique Gattefossé, No.90, (1997), S. 43-50). Diese polymeren Glyceride können aufgrund ihrer hohen HLB-Werte (z.B. Gelucire® 44/14 mit einem HLB-Wert von 14) als Tensid wirken. Wegen ihrer halbfesten Konsistenz müssen viele dieser Formulierungen in Gelatine-Kapseln abgefüllt werden. Dies gilt insbesondere für die Verwendung der in der Regel niedrig schmelzenden Glycerid-Tenside.
Die in DE 973 095 (D1) beschriebenen streufähigen Pulver enthalten wesentlich mehr Öl als Bindemittel.

Für die in DE 195 31 277 (D2) beschriebenen Schmelzextrudate werden bis zu 5 Gew.-% Lipid in Betracht gezogen. Als Lipide kommen Mono-, Di- und Triglyceride von natürlich vorkommenden Fettsäuren in Betracht, vorzugsweise hydriertes Rizinusöl, und vor allem Phospholipide, wobei hydrierte Lecithine, wie Soja- und Ei-Lecithin besonders bevorzugt sind. Hydriertes Rizinusöl besitzt einen Schmelzbereich von über 80 °C. Was mögliche Wirkstoffe angeht, so sind lediglich Verapamil-HCl und Nifedipin genannt.

Auch die DE 195 36 387 verweist auf einen möglichen Zusatz hochschmelzender Lipide zu Arzneiformen. Auch hier dient ein solcher Lipidzusatz als Formentrennmittel und Hilfsmittel zur Beeinflussung der Plastizität der Schmelze, also verfahrenstechnischen Zwecken bei der Herstellung der Arzneiform. Die beschriebenen Arzneiformen enthalten Vitamine.

Die in WO 00/00179 beschriebenen pulvrigen Zubereitungen schwer wasserlöslicher Wirkstoffe sollen zwar Öle, Fettsäuren oder Gemische davon enthalten können. Allgemeine Angaben zu Öl- oder Lipidmengen sind dieser Druckschrift aber nicht zu entnehmen. Auch wird zur Aufnahme des in Öl, Fettsäure oder Öl/Fettsäuregemisch dispergierten oder gelösten Wirkstoffs überwiegend Polyethylenglykol verwendet. Zwar betreffen auch vereinzelte Beispiele Formulierungen, die Hydroxypropylmethylcellulose, Polyvinylpyrrolidon oder (Meth)acrylharze enthalten können. In diesen Fällen liegt der Lipidgehalt aber unterhalb von 6 Gew.-%.

EP 0 570 327 betrifft Zusammensetzungen auf Polydextiose-Basis.
Die der vorliegenden Erfindung zugrunde liegende Aufgabe, selbstemulgierende Dosierungsformen zur Verfügung zu stellen, wird überraschenderweise durch Formulierungen gelöst, deren Formulierungsgrundlage eine Lipidkomponente und eine Bindemittelkomponente aufweist.

Gegenstand der vorliegenden Erfindung sind daher selbstemulgierende Formulierungen gemäß Patentanspruch 1 oder 15, sowie deren Verwendung gemäß Patentanspruch 22.

Die Formulierungen basieren auf
i) wenigstens einem Wirkstoff
   und einer Formulierungsgrundlage mit
ii) einer Lipidkomponente;
iii) einer Bindemittelkomponente; und
iv) gegebenenfalls weiteren Hilfsstoffen.

Der Begriff "Formulierung" meint im Rahmen der vorliegenden Erfindung ein aus den Komponenten i), ii), iii) und gegebenenfalls iv) zusammengesetztes Gemisch.

Unter Wirkstoffen im Sinne der Erfindung sind alle Stoffe mit einer physiologischen Wirkung zu verstehen. Es handelt sich insbesondere um pharmazeutische Wirkstoffe (für Mensch und Tier im human- wie auch tiermedizinischen Bereich), Wirkstoffe für die Pflanzenbehandlung, Insektizide, Futter- und Nahrungsmittelwirkstoffe, Riechstoffe, Aromen und Parfümöle. Die Wirkstoffmenge pro Dosiseinheit und die Konzentration können je nach Wirksamkeit und Freisetzungsgeschwindigkeit in weiten Grenzen variieren. Eine Bedingung ist, dass sie zur Erzielung der gewünschten Wirkung ausreichen.

Wirkstoffe im Sinne der Erfindung sind auch Vitamine und Mineralstoffe. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epoxiconazol und Quinmerac.

Zu den pharmazeutischen Wirkstoffen gehören z.B.:

Acebutolol, Acetylcystein, Acetylsalicylsäure, Acyclovir, Alprazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Selegilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Imipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Bromocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Folinsäure, Zidovudin.

Zu erfindungsgemäß verwendbaren Wirkstoffen gehört auch eine Vielzahl ätherischer Öle (Aetheroleum), wie Angelikaöl (Angelicae aetheroleum), Anisöl (Anisi aetheroleum), Arnicaöl (Arnicae aetheroleum), Aurantii aetheroleum, Baldrianöl (Valerianae aetheroleum), Basilici aetheroleum, Bergamoteöl (Bergamottae aetheroleum), Bohnenkrautöl, Bucco aetheroleum, Campher (Camphora), Cardamomi aetheroleum, Cassiaöl, Chenopodiumöl (Chenopodii aetheroleum), Chrysanthemenöl (Pyrethri aetheroleum), Cinae aetheroleum, Citronellöl, Citronenöl (Limonis aetheroleum), Citrusöl (Citri aetheroleum), Costusöl, Curcumaöl (Curcumae aetheroleum), Eberwurzöl (Carlinae aetheroleum), Elemiöl, Estragonöl, Eucalyptusöl (Eucalypti aetheroleum), Fenchelöl (Foeniculi aetheroleum), Fichtennadelöl (Piceae aetheroleum), Fichtenöl, Filicis aetheroleum, Galbanumöl, Gaultheriae aetheroleum, Geraniumöl, Guajakholzöl (Guaiaci aetheroleum), Haselwurzöl (Asari aetheroleum), Irisöl (Iridis aetheroleum), Johanniskrautöl (Hyperici aetheroleum), Kalmusöl, Kamillenöl (z.B. Chamomillae romanae aetheroleum; Matricariae aetheroleum), Kiefernnadelöl (Pini aetheroleum), Knoblauchöl (Allii sativi aetheroleum), Korianderöl (Coriandri aetheroleum), Kümmelöl (Carvi aetheroleum), Lauri aetheroleum, Lavendelöl (Lavandulae aetheroleum), Lemongrasöl, Liebstöckelöl (Levistici aetheroleum), Lorbeeröl, Lupuli strobuli aetheroleum, Macisöl, Majoranöl (Majoranae aetheroleum), Mandarinenöl, Melissenöl (Melissae aetheroleum; Calaminthae aetheroleum), Menthol (Mentholum), Millefolii aetheroleum, Minzöl (Menthae arvensis aetheroleum), Muskatellersalbeiöl, Muskatnußöl (Myristicae aetheroleum), Nardenöl (z.B. aus Nardostachys jatamansi), Nelkenöl (Caryophylli aetheroleum), Neroliöl, Niaouli, Olibanumöl, Ononidis aetheroleum, Opopranaxöl, Orangenöl, Oreganoöl, Orthosiphonöl, Patchouliöl, Petersilienöl (Petroselinum aetheroleum), Petitgrainöl, Pfefferminzöl (Menthae piperitae aetheroleum), Rainfarnöl (Tanaceti aetheroleum), Rosenholzöl, Rosenöl, Rosmarinöl (Rosmarini aetheroleum), Rutaöl (Rutae aetheroleum), Sabinae aetheroleum, Safranöl (Croci aetheroleum), Salbeiöl (Salviae aetheroleum), Sandelholzöl (Santali aetheroleum), Sassafrasöl (Sassafras aetheroleum), Sellerieöl (Apii aetheroleum), Senföl (Senapsis aetheroleum), Serphylli aetheroleum, Strohblumenöl (z.B. aus Helichrysum italicum), Tannenöl, Teebaum-öl, Terpentinöl (Terebinthinae aetheroleum), Thymianöl (Thymi aetheroleum), Wacholderbeeröl (Juniperi aetheroleum), Weihrauchöl, Ysopöl (z.B. aus Hyssopus officinalis var. decumbens), Zedernholzöl, Zimtöl (Cinnamomi aetheroleum), Zypressenöl.

Einige der zuvor genannten ätherischen Öle sind auch als Insektizide, z.B. Chrysanthemenöl und Kalmusöl, oder als Repellentia, z.B. Cassiaöl, Campher, Terpentinöl, Citronellöl, Zimtöl und Nelkenöl brauchbar.

Die ätherischen Öle stellen in der Regel Stoffgemische dar. Als Gemischkomponenten sind vor allem Terpen-Verbindungen, z.B. Monoterpene, Sesquiterpene sowie Biterpene und Triterpene, Phenylpropanderivate, einfache Phenole und deren Ether, Phenolcarbonsäuren, geradkettige Kohlenwasserstoffe und deren Derivate, kurzkettige Säuren, schwefelhaltige Verbindungen, z.B. Senföle, und stickstoffhaltige Substanzen, z.B. Indolderivate und Anthranilsäureester, zu nennen.

Im eigentlichen Sinne steht der Begriff "ätherisches Öl" für Substanzgemische, die durch Wasserdampfdestillation aus pflanzlichen Rohstoffen erhältlich sind. Die Gewinnung derartiger Öle kann je nach Menge und Art des Öls und in Abhängigkeit von dem zur Gewinnung eingesetzten pflanzlichen Rohstoff mit Ölextraktionsverfahren (Enfleurage-Verfahren), Lösungsmittelextraktion, Auspreßverfahren und anderen mechanischen Verfahren sowie Destillationsverfahren, zu denen auch die Wasserdampfdestillation gehört, erfolgen. Die so gewonnenen Öle können einer weiteren Reinigung unterzogen werden. Dies ist insbesondere bei wasserdampfdestillierten Ölen von Vorteil und insbesondere im Hinblick auf pharmazeutische oder kosmetische Anwendungen zweckmäßig.

Die Wirkstoffkomponente i) der erfindungsgemäßen Formulierungen enthält wenigstens einen Wirkstoff, insbesondere einen der zuvor genannten, und sie kann weitere, insbesondere unter den zuvor genannten auszuwählende, Wirkstoffe desselben oder eines anderen Typs enthalten.

Eine im Rahmen der vorliegenden Erfindung besondere Art von Wirkstoff ist aufgrund ihres fettähnlichen Charakters unter die Lipide, gegebenenfalls auch als Lipidderivat oder lipidhaltiges Gemisch, zu fassen. Derartige Wirkstoffe werden im folgenden als lipidartige Wirkstoffe bezeichnet. Ausführungen zu Lipiden beziehen sich auch auf derartige lipidartige Wirkstoffe. Insbesondere handelt es sich bei den erfindungsgemäß brauchbaren lipidartigen Wirkstoffen um Öle und vor allem um die zuvor genannten ätherischen Öle. Diese Art von Wirkstoff kann einen Teil oder die Gesamtheit sowohl der Wirkstoffkomponente i) als auch der Lipidkomponente ii) bilden. Gemäß eines besonderen Aspekts betrifft die vorliegende Erfindung daher selbstemulgierende Formulierungen auf Basis
i') wenigstens eines lipidartigen Wirkstoffs und gegebenenfalls weiterer Wirkstoffe
   und einer Formulierungsgrundlage mit
ii') gegebenenfalls einem weiteren Lipidkomponentenanteil;
iii) einer Bindemittelkomponente; und
iv) gegebenenfalls weiteren Hilfsstoffen.

Demnach beinhaltet die Komponente i') die Wirkstoffkomponente i) sowie wenigstens einen Teil der Lipidkomponente ii); und die Komponente ii') den Teil der Lipidkomponente ii), der nicht von i') umfasst ist. Gemäß einer speziellen Ausführungsform dieses Aspekts umfaßt die Komponente i') die Wirkstoffkomponente i) sowie die Lipidkomponente ii), mit der Folge das die Komponente ii') nicht vorhanden ist. Die Komponente i') umfaßt wenigstens einen lipidartigen Wirkstoff, d.h. sie kann auch 2 oder mehrere dieser lipidartigen Wirkstoffe bzw. auch einen oder mehrere weitere Wirkstoffe umfassen. Gemäß einer weiteren speziellen Ausführungsform dieses Aspekts besteht die Komponente i') aus wenigstens einem lipidartigen Wirkstoff.

Demnach bezieht sich der Begriff "Lipidkomponente" - sofern nicht anderes angegeben ist - im allgemeinen auf die Komponente ii) und im speziellen z.B. auf den Lipidanteil der Komponente i') gegebenenfalls in Kombination mit der Komponente ii').

Vorteilhaft sind die erfindungsgemäßen Formulierungen insbesondere für diejenigen Wirkstoffe, die von der solubilisierenden Eigenschaft der bei Auflösung der Formulierungen in wässrigen Medien resultierenden Emulsionen profitieren. Dies sind vor allem schwer lösliche Wirkstoffe, insbesondere solche, bei denen zum Lösen 1 Teils Wirkstoff wenigstens 100, insbesondere wenigstens 1000 Teile und vorzugsweise wenigstens 10000 Teile Wasser erforderlich sind, aber auch leicht wasserlösliche Wirkstoffe, die nur ungenügende Wirkungen bei bestimmten Verabfolgungsarten entfalten.

Die Wirkstoffkomponente i) macht 0,1 bis 50 Gew.-%, vorzugsweise 1 bis 30 Gew.-% und insbesondere 5 bis 20 Gew.-% der Formulierung aus. Angaben in Gew.-% beziehen sich, sofern nicht anderes angegeben ist, auf das Gesamtgewicht der Formulierung.

Die Formulierungsgrundlage erfindungsgemäßer Formulierungen enthält Hilfsstoffe, nämlich gemäß einer Ausführungsform wenigstens ein Lipid, wenigstens ein Bindemittel und gegebenenfalls weitere Hilfsstoffe und gemäß einer weiteren Ausführungsform, bei der die Wirkstoffkomponente wenigstens einen lipidartigen Wirkstoff enthält, wenigstens ein Bindemittel und gegebenenfalls weitere Lipide und/oder weitere Hilfsstoffe.

Die Lipidkomponente erfindungsgemäßer fester Formulierungen enthält wenigstens ein Lipid, womit auch Lipidderivate und lipidhaltige Gemische bezeichnet sein sollen.

Der Begriff Lipid steht als Sammelbezeichnung für Fette und fettähnliche Substanzen. Die Fettähnlichkeit definiert sich insbesondere über das Löslichkeitsverhalten. Demnach sind fettähnliche Substanzen wie Fette selbst z.B. in Wasser praktisch unlöslich. Wasserunlöslich sind Substanzen im erfindungsgemäßen Sinne insbesondere dann, wenn zum Lösen von 1 Teil Substanz wenigstens 1000 bis 10000 Teile und vorzugsweise wenigstens 10000 Teile Wasser erforderlich sind. Man bezeichnet sie auch als lipophil bzw. hydrophob.

Gemäß einer Ausführungsform der vorliegenden Erfindung sind Lipide bevorzugt, die sich ein Organismus zu eigen machen kann, also beispielsweise aufnehmen und gegebenenfalls metabolisieren kann. In diesem Sinne realisieren diejenigen Lipide und Lipidderivate, die über den Gastrointestinaltrakt aufgenommen werden können, eine besondere Ausführungsform der vorliegenden Erfindung insbesondere im Rahmen pharmazeutischer Anwendungen. Natürliche Lipide und Derivate natürlicher Lipide, die pflanzlichen oder tierischen Ursprungs sein können, sind bevorzugt.

Insbesondere im Rahmen pharmazeutischer Anwendungen ist wenigstens ein Lipid der Lipidkomponente bevorzugt ausgewählt unter körpereigenen Lipiden. Zu den körpereigenen Lipiden gehören vor allem Lipide auf Basis von Fettsäuren mit einer geradzahligen Anzahl an Kohlenstoffatomen, insbesondere entsprechende Glyceride und Fettsäuren bzw. Derivate davon.

Der Begriff Fettsäure bezeichnet eine Gruppe aliphatischer gesättigter oder ungesättigter Carbonsäuren. In der Regel sind es unverzweigte Ketten mit 6 bis 30, vorzugsweise 8 bis 22 und insbesondere 8 bis 18 Kohlenstoffatomen. Zu den gesättigten Fettsäuren gehören beispielsweise Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure. Die ungesättigten Fettsäuren können einfach oder mehrfach ungesättigt, insbesondere einfach, zweifach, dreifach, vierfach, fünffach oder sechsfach ungesättigt sein. Beispielsweise gehören zu den einfach ungesättigten Fettsäuren Palmitoleinsäure, Ölsäure und Erucasäure, zu den zweifach ungesättigten Fettsäuren Sorbinsäure und Linolsäure, zu den dreifach ungesättigten Fettsäuren Linolensäure und Elaeostearinsäure, zu den vierfach ungesättigten Fettsäuren Arachidonsäure, zu den fünffach ungesättigten Fettsäuren Clupanodonsäure und zu den sechsfach ungesättigten Fettsäuren Docosahexaensäure. Bevorzugt sind einfach oder mehrfach ungesättigte Fettsäuren, vor allem Ölsäure, Palmitoleinsäure, Erucasäure, Linolsäure, Linolensäure.

Mit dem Begriff Glyceride bezeichnet man Ester des Glycerins. Je nach Anzahl der Estergruppen spricht man von Mono-, Di- und Triglyceriden. Der Säurerest eines Monoglycerids kann an 1- oder 2-Position sitzen und die Säurereste von Di- und Triglyceriden können gleich oder verschieden sein und in jeder erdenklichen Art auf die drei möglichen Positionen des Glycerins verteilt sein. Bei den Säureresten handelt es sich bevorzugt um die zuvor beschriebenen Fettsäuren. Beispielsweise gehören zu den Monoglyceriden Glycerinmonobehenat, Glycerinmonocaprat, Glycerinmonococoat, Glycerinmonoerucat, Glycerinmonoisostearat, Glycerinmonolanolat, Glycerinmonolaurat, Glycerinmonolinoleat, Glycerinmonomyristat, Glycerinmonooleat, Glycerinmonopalmitat, Glycerinmonoricinoleat, Glycerinmonostearat, zu den Diglyceriden Glycerindicaprylat, Glycerindilaurat, Glycerindimyristat, Glycerindioleat, Glycerindipalmitat und Glycerindistearat, zu den Triglyceriden Glycerintricaprylat, Glycerintrilaurat, Glycerintrimyristat, Glycerintrioctanoat, Glycerintrioleat, Glycerintriricinoleat und Glycerintristearat.

Bevorzugt sind Mono-, Di- und Triglyceride mit ungesättigten Fettsäureresten, insbesondere den erfindungsgemäß bevorzugt verwendbaren Fettsäureresten, vor allem Glycerinmonooleat, Glycerindioleat, Glycerintrioleat.

Vorzugsweise enthält die Lipidkomponente erfindungsgemäßer Formulierungen wenigstens eines der zuvor beschriebenen Lipide oder ein Gemisch aus wenigstens zwei der zuvor beschriebenen Lipide, wobei sie weitere dieser Lipide und auch andere Lipide enthalten kann.

Gemäß einer Ausführungsform der vorliegenden Erfindung besteht die Lipidkomponente aus einem der zuvor beschriebenen Lipide.

Gemäß einer anderen Ausführungsform der vorliegenden Erfindung besteht die Lipidkomponente aus einem Lipidgemisch aus wenigstens zwei der zuvor beschriebenen Lipide, insbesondere aus einem Fettsäuregemisch, einem Glyceridgemisch oder einem Fettsäure/Glycerid-Gemisch.

Zu den Derivaten natürlicher Lipide, die pflanzlichen oder tierischen Ursprungs sein können, gehören vor allem diejenigen natürlichen Lipide, die chemisch und/oder physikalisch behandelt sind. Eine geeignete chemische Behandlung ist beispielsweise die Hydrierung ungesättigter Fettsäuren oder Fettsäurereste in Glyceriden. Eine geeignete physikalische Behandlung ist beispielsweise die Fraktionierung natürlicher Lipidgemische.

Zu den erfindungsgemäß verwendbaren Lipiden gehören auch lipidhaltige Naturstoffextrakte, die neben Lipid auch weitere Bestandteile enthalten können. Zu nennen sind hier vor allem die in einschlägigen Arzneibüchern gelisteten Lipide und Lipidgemische und auch Derivate davon, wie pflanzliche Öle oder tierische Fette, z.B. Olivenöl, Rizinusöl, Sesamöl, Erdnußöl, Mandelöl, Leinöl, Kakaobutter, Saffloröl, mittelkettige Triglyceride (Triglycerida mediocatenalia), Calcii behenas, Glyceroli monostearas, mittelkettige Partialglyceride (Partialglycerida mediocatenalia), höherkettige Partialglyceride (Partialglycerida longiaatenalia), die gegebenenfalls auch hydriert oder raffiniert sein können, wie hydriertes Rizinusöl bzw. raffiniertes Rizinusöl. Auch hier sind Lipide mit einem Gehalt an ungesättigten Fettsäuren bzw. Fettsäureresten,bevorzugt.

Gemäß einer besonderen Ausführungsform der vorliegenden Erfindung wird wenigstens ein Teil der Lipidkomponente von wenigstens einem lipidartigen Wirkstoff gebildet. Auf obige Ausführungen zu lipidartigen Wirkstoffen wird sinngemäß Bezug genommen. Eine spezielle Ausführungsform sind erfindungsgemäße Formulierungen mit einer Lipidkomponente, die aus wenigstens einem lipidartigen Wirkstoff, insbesondere den zuvor genannten Ölen und vor allem den ätherischen Ölen, besteht.

Gemäß einer besonderen Ausführungsform weist die Lipidkomponente ii) bzw. der Lipidanteil der Komponente i') gegebenenfalls in Kombination mit der Komponente ii') einen HLB-Wert von höchstens 12, vorzugsweise von höchstens 8 und insbesondere von höchstens 5 auf. Das HLB-System (Hydrophil-Lipophil-Balance system) ordnet grenzflächenaktiven Stoffen numerische Zahlenwerte zu, lipophile Substanzen erhalten niedrige, hydrophile höhere HLB-Werte (Fiedler, H.B., Lexikon der Hilfsstoffe für Pharmazie, Kosmetik, und angrenzende Gebiete, 4.Aufl., Aulendorf: ECV-Editio-Cantor-Verlag (1996)). Insbesondere ist die Lipidkomponenete ii) bzw. der Lipidanteil der Komponente i') gegebenenfalls in Kombination mit der Komponente ii') nicht oder nur schlecht in Wasser löslich. Demnach läßt sich diese Ausführungsform vor allem mit den zuvor genannten Fettsäuren und Glyceriden bzw. Ölen und insbesondere ätherischen Ölen verwirklichen.

Gemäß einer weiteren bevorzugten Ausführungsform weist die Lipidkomponente ii) bzw. der Lipidanteil der Komponente i') gegebenenfalls in Kombination mit der Komponente ii') einen Schmelzpunkt von höchstens 50°C, vorzugsweise von höchstens 40°C und insbesondere von weniger als 30°C auf. Demnach läßt sich diese Ausführungsform vor allem mit Fettsäuren wie Tridecansäure, Laurinsäure, Elaeostearinsäure, vorzugsweise Undecansäure, Caprinsäure, Erucasäure, insbesondere Pelargonsäure, Caprylsäure, Önanthsäure, Capronsäure, Isostearinsäure, Ölsäure, Palmitoleinsäure, Linolsäure, Linolensäure, Arachidonsäure, Clupanodonsäure und Docosahexaensäure, und Glyceriden, wie Glycerinmonolaurat, Glycerinmonolinoleat, Glycerinmonooleat, Glycerinmonopalmitat, Glycerinmonoricinoleat, Glycerindioleat, Glycerintrioleat und Glycerintriricinoleat, bzw. mit den zuvor genannten ätherischen Ölen verwirklichen.

Insbesondere bevorzugt ist es, dass wenigstens ein Teil der Lipidkomponente ii) bzw. des Lipidanteils der Komponente i') gegebenenfalls in Kombination mit der Komponente ii') und wenigstens ein Teil der Bindemittelkomponente in den erfindungsgemäßen Formulierungen eine molekulardisperse Verteilung ausbilden. Ist der Lipidanteil größer als der Bindemittelanteil, so spricht man von einer molekulardispersen Verteilung des Bindemittels im Lipid. Vorzugsweise ist der Lipidanteil geringer als der Bindemittelanteil; dann spricht man von einer molekülardispersen Verteilung des Lipids im Bindemittel.

Der Begriff "molekulardispers" ist dem Fachmann bekannt und beschreibt im Wesentlichen Systeme, in denen eine Substanz, im vorliegenden Fall wenigstens ein Teil und vorzugsweise der überwiegende Teil der Lipid- bzw. Bindemittelkoinponente, in einem Lösungsmittel in homogener Verteilung dispergiert ist. Das Lösungsmittel bildet dabei in der Regel eine Matrix, die erfindungsgemäß von der Bindemittel- bzw. Lipidkomponente oder zumindest von einem überwiegenden Teil von der Bindemittel- bzw. Lipidkomponente gebildet wird. Der Anteil an Lipidkristallen in einer erfindungsgemäßen Formulierung liegt in der Regel unter 12% und insbesondere unter 5%. Angaben zu Kristallanteilen beziehen sich auf die Gesamtmenge der jeweiligen Komponente.

Gemäß einer besonderen Ausführungsform sind molekulardisperse Systeme fest, sie werden dann als feste Lösungen bezeichnet.

Eine erfindungsgemäße Formulierung, die im Wesentlichen frei von Lipidkristallen ist, stellt eine besondere Ausführungsform der vorliegenden Erfindung dar. Dieser Zustand entspricht der maximal möglichen Homogenisierung des Lipids bzw. Bindemittels in der Matrix. In der molekulardispersen Verteilung ist das System grenzflächenfrei.

Gemäß einer weiteren besonderen Ausführungsform liegt wenigstens ein Teil der Wirkstoffkomponente als molekulardisperse Verteilung vor. Der Anteil an Wirkstoffkristallen in einer erfindungsgemäßen Formulierung liegt unter 5%. Zu diesen Formulierungen gehören insbesondere diejenigen, die im Wesentlichen frei von Wirkstoffkristallen sind. Dieser Zustand entspricht der maximal möglichen Homogenisierung des Wirkstoffs in der Formulierungsgrundlage.

Erfindungsgemäße Formulierungen, die im Wesentlichen frei von Lipid- und Wirkstoffkristallen sind, und vor allem solche, in denen im Wesentlichen kein Bestandteil kristalline Anteile aufweist (im wesentlichen amorphe bzw. kristallfreie Formulierungen), stellen eine weitere besondere Ausführungsform der vorliegenden Erfindung dar. Dieser Zustand entspricht der maximal möglichen Homogenisierung der Formulierungskomponenten. In der molekulardispersen Verteilung ist die Formulierung grenzflächenfrei.

Der Zustand solcher molekulardisperser Verteilungen, insbesondere der fester Lösungen, läßt sich mit bekannten analytischen Verfahren untersuchen, z.B. mit der Differential Scanning Calorimetry (DSC) oder mit Weitwinkelröntgenstreuung-Messungen (WAXS-Messungen). Liegt eine molekulardisperse vor, so fehlt der bei der DSC-analytischen Messung der kristallinen Reinsubstanz auftretende, in der Regel endotherme Schmelzpeak. Eine weitere Möglichkeit zur Kennzeichnung einer molekulardispersen Verteilung ist die Intensitätsverminderung und/oder Abwesenheit typischer Röntgenbeugungs-Signale bei WAXS-Analytik.

Der Anteil der Lipidkomponente an der Formulierung beträgt 6 bis 60 Gew.-%, vorzugsweise 11 bis 40 Gew.-% und insbesondere 16 bis 25 Gew.-%.

Ein Kriterium für die Ermittlung der optimalen Menge Lipid ist die Homogenität der erfindungsgemäßen Formulierung in der Schmelze. Insbesondere mit Blick auf die Obergrenze sollte eine homogene Einarbeitung des Lipids in die Schmelze ohne Phasenseparation gewährleistet sein.

Gemäß einer besonderen Ausführungsform der vorliegenden Erfindung beträgt der Anteil der Lipidkomponente ii) bzw. der Lipidanteil der Komponente i'), gegebenenfalls in Kombination mit der Komponente ii'), bezogen auf die Bindemittelkomponente höchstens 40 Gew.-%, vorzugsweise höchstens 30 Gew.-% und insbesondere höchstens 25 Gew.-%.

Die Bindemittelkomponente der erfindungsgemäßen Formulierungen kann auch als Bindemittel begriffen werden, das zumindest teilweise eine Bindemittelmatrix, insbesondere eine Polymermatrix bildet. Bindemittel im Sinne der Erfindung sind feste schmelzbare Lösungsmittel. Die Bindemittelmatrix dient vor allem zur Aufnahme, insbesondere zum Lösen wenigstens eines Teils der Lipidkomponente ii) bzw. des Lipidanteils der Komponente i') gegebenenfalls in Kombination mit der Komponente ii'). Vorzugsweise kommt es dabei zur Ausbildung von molekulardispersen Verteilungen. Diesbezüglich wird auf obige Ausführungen in Zusammenhang mit der Lipidkomponente Bezug genommen.

Vorzugsweise ist die Bindemittelkomponente in wässrigen Medien, zweckmäßigerweise unter den Anwendungsbedingungen, also insbesondere physiologischen Bedingungen, zumindest teilweise löslich oder quellbar.

Im Rahmen der vorliegenden Beschreibung gehören zu wäßrigen Medien Wasser und Gemische aus Wasser und weiteren Komponenten, die mindestens 50 Gew.-%, bevorzugt mindestens 70 Gew.-% und insbesondere mindestens 90 Gew.-% Wasser enthalten. Zu wäßrigen Medien gehören vor allem Körperflüssigkeiten, wie Flüssigkeiten des Verdauungstraktes, z.B. Magensaft und Darmsäfte, Speichel, Urin, Fäcesflüssigkeit, Wundsekret, Ergüsse, Fruchtwasser, Punktate, Lymphe und Blut; Getränke auf Wasserbasis wie Tee, Kaffee, Limonaden oder Babynahrung, Nahrung zur parenteralen Ernährung, Säfte, Sirupe, Brauchwasser, z.B. Wasser zum Einsatz in der Tierfütterung und zum Gießen von Pflanzen sowie zur Schädlingsbekämpfung, Wasser zum Einsatz in Reinigungsprozessen, z.B. zum Waschen von Textilien oder Geschirr, Badewasser, wäßrige Vehikel zur Verwendung in Formulierungen im Pharma-, Kosmetik- oder Pflanzenbehandlungsbereich, z.B. parenteral verabreichbare Vehikel, Salben-, Creme-, Pasten- oder Gelgrundlagen, Wasser oder wäßrige Medien zur Verwendung in Aromatherapien oder zur Inhalation.

Unter Quellung versteht man im Wesentlichen einen Vorgang, bei dem sich Volumen und/oder Gestalt eines Festkörpers, beispielsweise einer erfindungsgemäßen festen Formulierung, bei Einwirkung von Flüssigkeiten, Dämpfen und Gasen, ändern. Quellbar bzw. löslich sind vor allem hydrophile Polymere, die Wasser zumindest oberflächlich anzulagern und/oder zwischen die Polymerketten, vornehmlich durch Absorption, aufzunehmen vermögen. Eine begrenzte Quellung führt in der Regel zur Gelbildung, weshalb erfindungsgemäß brauchbare begrenzt quellbare Polymere unter den gemeinhin als Gelbildnern bekannten Polymeren ausgewählt werden können. Eine unbegrenzte Quellung führt in der Regel zur Ausbildung von Lösungen oder kolloidalen Lösungen, weshalb erfindungsgemäß brauchbare, unbegrenzt quellbare Polymere unter den im jeweiligen wässrigen Medium zumindest kolloidal löslichen Polymeren ausgewählt werden können. Für den Bereich der pharmzeutischen Anwendungen und insbesondere mit Blick auf Körperflüssigkeiten, beispielsweise denen des Gastrointestinaltraktes, ist zu berücksichtigen, dass die physiologischen Bedingungen, vor allem der pH-Wert, örtlich unterschiedlich sein können. Ist es beispielsweise bevorzugt, dass der Wirkstoff vornehmlich im Duodenum aufgenommen wird, so kann es von Vorteil sein, daß die Bindemittelkomponente unter den im Duodenum herrschenden Bedingungen quellbar ist. Insbesondere kann es von Vorteil sein, wenn in vorgeschalteten Abschnitten des Gastrointestinaltraktes, insbesondere im Magen nur eine geringe oder vorzugsweise im Wesentlichen keine Quellung erfolgt. Es sei allerdings schon an dieser Stelle bemerkt, dass ein solches Verhalten erfindungsgemäßer Formulierungen nach Verabfolgung auch mit anderen Mitteln gewährleistet werden kann, im vorstehend geschilderten Fall beispielsweise mit magensaftresistenten Überzügen oder mehrschichtigen Formulierungen, in denen meist innenliegende wirkstoffhaltige Schichten erst am erwünschten Ort einer Quellung oder Lösung ausgesetzt sind.

Gemäß einer besonderen Ausführungsform bildet die Bindemittelkomponente iii) unter den Anwendungsbedingungen der Formulierung keine Micellen aus. Eine CMC (kritische Micellbildungskonzentration) wird nicht erreicht.

Verfahrenstechnisch sind Bindemittelkomponenten vorteilhaft, die thermoplastisch verarbeitbar sind.

Vorzugsweise ist wenigstens ein Bindemittel der Bindemittelkomponente ausgewählt unter:
Synthetischen Polymeren, wie Polyvinyllactamen, insbesondere Polyvinylpyrrolidon (PVP); Copolymeren von Vinyllactamen, wie N-Vinylpyrrolidon, N-Vinylpiperidon und N-Vinyl-ε-caprolactam, aber insbesondere N-Vinylpyrrolidon, mit (Meth)acrylsäure und/oder (Meth)acrylsäureestern, wie langkettigen (Meth)acrylaten, z.B. Stearyl(meth)acrylat, Dialkylaminoalkyl(meth)acrylaten, die quaternisiert sein können, und Maleinsäureanhydrid, Vinylestern, insbesondere Vinylacetat, Vinylformamid, Vinylsulfonsäure oder quaternisiertem Vinylimidazol; Copolymerisaten von Vinylacetat und Crotonsäure; teilverseiftem Polyvinylacetat; Polyvinylalkohol; (Meth)acrylharzen, wie Polyhydroxyalkyl(meth)acrylaten, Poly(meth)acrylaten, Acrylatcopolymeren, z.B. aus Acrylsäurealkylestern mit (Meth)acrylsäure, und Copolymerisaten von Dimethylaminoethylacrylaten und Methacrylestern (z.B. Eudragit-Typen); Polyalkylenglykolen, wie Polypropylenglykolen und Polyethylenglykolen, vorzugsweise mit Molekulargewichten oberhalb von 1000, besonders bevorzugt oberhalb von 2000 und ganz besonders bevorzugt oberhalb von 4000 (z.B. Polyethylenglykol 6000); Polyalkylenoxiden, wie Polypropylenoxiden und vor allem Polyethylenoxiden, vorzugsweise hochmolekular, vor allem mit gewichtsmittleren Molekulargewichten von mehr als 100000; Copolymerisaten von Methylmethacrylat und Acrylsäure; Polyacrylamiden, Polyvinylformamid (gegebenenfalls partiell oder vollständig hydrolysiert);
modifizierten natürlichen Polymeren, z.B. modifizierten Stärken und modifizierten Cellulosen, wie Celluloseestern und bevorzugt Celluloseethern, z.B. Methylcellulose und Ethylcellulose, Hydroxyalkylcellulosen, insbesondere Hydroxypropylcellulose, Hydroxyalkyl-Alkylcellulosen, insbesondere HydroxypropylMethylcellulose oder Hydroxypropyl-Ethylcellulose, Cellulosephthalaten, insbesondere Celluloseacetatphthalat und Hydroxypropylmethylcellulosephthalat, Stärkeabbauprodukte, insbesondere Stärkeverzuckerungsprodukte, wie Maltodextrin;
natürlichen oder überwiegend natürlichen Polymeren, wie Gelatine, Polyhydroxyalkanoaten, z.B. Polyhydroxybuttersäure und Polymilchsäure, Polyaminosäuren, z.B. Polylysin, Polyasparagin, Polydioxane und Polypeptiden, und Mannanen, insbesondere Galactomannanen; und
nichtpolymeren Bindemitteln wie Polyolen, beispielsweise den in WO 98/22094 und EP 0 435 450 beschriebenen, insbesondere Zuckeralkoholen, wie Maltit, Mannit, Sorbit, Cellobiit, Lactit, Xylit, Erythrit und Isomalt (Palatinit).

Gemäß einer Ausführungsform der vorliegenden Erfindung ist wenigstens ein Bindemittel der Bindemittelkomponente ausgewählt unter den modifizierten natürlichen und vor allem den synthetischen Polymeren. Gemäß einer weiteren Ausführungsform der vorliegenden Erfindung ist wenigstens ein Bindemittel der Bindemittelkomponente ausgewählt unter den Zuckeralkoholen oder den Stärkeverzukkerungsprodukten.

Insbesondere bevorzugt ist wenigstens ein Polymer der Bindemittelkomponente ausgewählt unter Polyvinylpyrrolidonen, Vinylpyrrolidon-Vinylacetat-Copolymeren, Hydroxyalkylcellulosen, Hydroxyalkyl-Alkylcellulosen, Cellulosephthalaten, Polyalkylenglycolen, (Meth)acrylharzen: beispielsweise die unter der Handelsbezeichnung Kollidon® geführten Polyvinylpyrrolidone mit gewichtsmittleren Molekulargewichten von etwa 2000 bis etwa 1,5 x 10⁶, beispielsweise das unter der Handelsbezeichnung Kollidon® 17 PF geführte Polyvinylpyrrolidon mit einem gewichtsmittleren Molekulargewicht von etwa 7000 bis etwa 11000; Vinylpyrrolidon-Vinylacetat-Copolymere insbesondere mit einem Verhältnis Vinylpyrrolidon:Vinylacetat von etwa 30 zu etwa 70 bis etwa 70 zu etwa 30, beispielsweise das unter der Handelsbezeichnung Kollidon® VA 64 geführte Produkt mit einem Verhältnis Vinylpyrrolidon:Vinylacetat von etwa 60 zu etwa 40; Hydroxyalkylcellulosen mit 1 bis 3 Kohlenstoffatomen im Alkylteil, insbesondere Hydroxypropylcellulose, beispielsweise die unter der Handelsbezeichnung Klucel® geführte Hydroxypropylcellulose; Hydroxyalkyl-Alkylcellulosen mit 1 bis 3 Kohlenstoffatomen in den Alkylteilen, insbesondere Hydroxypropylmethylcellulose, beispielsweise die unter der Handelsbezeichnung Methocel® geführten Ethyl-, Hydroxyethyl-, Hydroxypropyl und Carboxymethylethergruppen enthaltenden Methylcellulose- und Methylcellulosederivatgemische, Cellulosephthalate, insbesondere Hydroxypropylmethylcellulosephthalat, Polyalkylenglycole mit 2 und/oder 3 Kohlenstoffatomen im Alkylenteil, insbesondere Polyethylenglycole, beispielsweise die unter der Handelsbezeichnung Lutrol® geführten Polyethylenglycole mit gewichtsmittleren Molekulargewichten von 2000 bis etwa 20000, und Polypropylenglycole, Copolymerisate auf Basis von Dimethylaminoethylmethacrylat und Methacrylsäureestern wie Methacrylsäuremethylester und Methacrylsäurebutylester, beispielsweise die unter der Handelsbezeichnung Eudragit® E geführten Acrylharze auf Basis von Dimethylaminoethylmethacrylat, Methyl- und Butyl(meth)acrylat mit gewichtsmitt-, leren Molekulargewichten von etwa 150000, Copolymerisate mit anionischem Charakter auf Basis von Methacrylsäure und Methacrylsäuremethylester, beispielsweise die unter den Handelsbezeichnungen Eudragit® L bzw. S geführten Acrylharze mit gewichtsmittleren Molekulargewichten von etwa 250000 bzw. 135000.

Ganz besonders bevorzugt sind die vorstehend genannten Polyvinylpyrrolidone und Cellulosederivate, vor allem Kollidon® VA 64 und niedermolekulare Hydroxypropylcellulose, z.B. Rlucel®EF mit gewichtsmittleren Molekulargewichten von etwa 45000 bis etwa 70000 bzw. etwa 80000, und Hydroxypropylmethylcellulose, z.B. Methocel® E3, E5 und E7.

Die Bindemittelkomponente erfindungsgemäßer Formulierungen enthält vorzugsweise wenigstens eines der zuvor beschriebenen Bindemittel. Sie kann weitere dieser Bindemittel und/oder andere Bindemittel enthalten. Über die Art des gewählten Bindemittels oder der Abmischung unterschiedlicher Bindemittele können die Eigenschaften der erfindungsgemäßen Formulierung variiert werden. Insbesondere kann auf diese Weise die Wirkstoff-Freisetzung gesteuert werden.

Gemäß einer Ausführungsform der vorliegenden Erfindung besteht die Bindemittelkomponente aus einem der zuvor beschriebenen Bindemittel. Gemäß einer anderen Ausführungsform der vorliegenden Erfindung besteht die Bindemittelkomponente aus einem Gemisch aus wenigstens zwei der zuvor beschriebenen Bindemittel.

Gemäß einer speziellen Ausführungsform der vorliegenden Erfindung enthält die Bindemittelkomponente wenigstens einen Zuckeralkohol, der bevorzugt ausgewählt ist unter Maltit, Xylit und Isomalt, und/oder ein oder mehrere Stärkeverzuckerungsprodukt(e), insbesondere Maltodextrin, gegebenenfalls in Kombination mit einem oder mehreren hydrophilen Polymeren, die bevorzugt ausgewählt sind unter den oben genannten modifizierten natürlichen und synthetischen Polymeren, z.B. Polyvinylpyrrolidonen, Vinylpyrrolidon-Copolymerisaten insbesondere mit Vinylacetat, oder Cellulosederivaten, insbesondere Hydroxypropylcellulosen, Hydroxypropylmethylcellulosen oder Methylcellulosen, oder Polyethylenglykolen. Diese Bestandteile dieser Bindemittelkomponente liegen bevorzugt in folgenden Mengenverhältnissen vor:
iii1) 5 bis 90 Gew.-%, vorzugsweise 10 bis 50 Gew.-% und insbesondere 15 bis 30 Gew.-% wenigstens eines Zuckeralkohols und/oder wenigstens eines Stärkeverzuckerungsproduktes;
iii2) 10 bis 95 Gew.-%, vorzugsweise 50 bis 90 Gew.-% und insbesondere 70 bis 85 Gew.-% wenigstens eines hydrophilen Polymers;
iii3) gegebenenfalls wenigstens eines der oben beschriebenen Polymere;
wobei die Summe der Anteile iii1), iii2), und iii3) 100 Gew.-% der Bindemittelkomponente beträgt.

Vorteilhaft für die Verwendung als polymeres Bindemittel sind solche Bindemittel, die einen K-Wert (nach H. Fikentscher, Cellulose-Chemie 13 (1932), S. 58-64 und 71-74) im Bereich zwischen 10 und 100, insbesondere zwischen 15 und 80 aufweisen.

Der Anteil der Bindemittelkomponente an der erfindungsgemäßen Formulierung beträgt in der Regel 20 bis 93,9 Gew.-%, vorzugsweise 30 bis 90 Gew.-% und insbesondere 40 bis 80 Gew.-%.

Im Bereich der pharmazeutischen Formulierungen beträgt der Anteil der Bindemittelkomponente an der erfindungsgemäßen Formulierung insbesondere 20 bis 80 Gew.-%, vorzugsweise 30 bis 60 Gew.-% und insbesondere 40 bis 50 Gew.-%.

Erfindungsgemäße Formulierungen können neben Lipidkomponente ii) bzw. dem Lipidanteil der Komponente i') gegebenenfalls in Kombination mit der Komponente ii'), und Bindemittelkomponente iii) weitere Hilfstsoffe, z.B. pharmazeutisch und kosmetisch akzeptable Hilfsstoffe enthalten (Hilfsstoffkomponente iv). Solche Hilfsstoffe können die Herstellung der Formulierung erleichtern und/oder deren Eigenschaften modulieren. Art und Menge werden vorteilhafterweise so gewählt, daß sie die Ausbildung der speziellen Eigenschaften der erfindungsgemäßen Formulierungen und einer gegebenenfalls vorhandenen molekulardispersen Verteilung, insbesondere einer festen Lösung nicht beinträchtigen bzw. nicht zu einer Destabilisierung dieses Systems beitragen.

Hilfsstoffe sind z.B. übliche galenische Hilfsstoffe, deren Gesamtmenge bis zu 100 Gew.-%, bezogen auf die Bindemittelkomponente, betragen kann, z.B.
Füllstoffe, wie die oben erwähnten Zuckeralkohole, z.B. Mannit, Sorbit, Xylit und Isomalt (vgl. DE 195 36 394), Talkum, Saccharose, Lactose, Getreide- oder Maisstärke, Kartoffelmehl, soweit vorhanden insbesondere in einer Konzentration von 0,02 bis 50, vorzugsweise 0,20 bis 20 Gew.-%, bezogen auf das Gesamtgewicht des Gemisches;
Schmiermittel, Gleitmittel und Trennmittel wie Magnesium-, Aluminium- und Calciumstearat, Talkum und Silicone, sowie tierische oder pflanzliche Fette, insbesondere in hydrierter Form und solche, die bei Raumtemperatur fest sind. Diese Fette haben vorzugsweise einen Schmelzpunkt von 30°C oder höher. Verfahrenstechnisch im Hinblick auf die Schmelzextrusion bevorzugt sind - wie in der DE 197 31 277 beschrieben - Triglyceride der C₁₂-, C₁₄-, C₁₆- und C₁₈-Fettsäuren, oder - zwecks Verbesserung der Verarbeitungseigenschaften - Lecithin, wie im Zusammenhang mit der Extrusion einer Isomalt enthaltenden Polymer-Wirkstoffschmelze in der DE 195 36 394 beschrieben ist. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. In der Regel erfüllen die erfindungsgemäß vorhandenen Lipide die Funktion dieser Hilfsstoffe, so daß nur geringe Mengen und vorteilhafterweise keine Schmiermittel, Gleitmittel und Trennmittel als Hilfsstoffe der Formulierung zugesetzt werden. Soweit vorhanden, beträgt die Gesamthilfsstoffmenge an Schmier- und Trennmitteln vorzugsweise 0,1 bis 10 Gew.-% und insbesondere 0,1 bis 1 Gew.% bezogen auf das Gesamtgewicht des Gemisches;
Fließmittel, z.B. Kieselerden, insbesondere die unter der Handelsbezeichnung Aerosil® geführten hochreinen Siliziumdioxide, soweit vorhanden insbesondere in einer Menge von 0,1 bis 5 Gew.-%, bezogen auf das Gesamtgewicht des Gemisches;
Farbstoffe, wie Azofarbstoffe, organische oder anorganische Pigmente oder Farbstoffe natürlicher Herkunft, wobei anorganische Pigmente soweit vorhanden in einer Konzentration von 0,001 bis 10, vorzugsweise 0,5 bis 3 Gew.-%, bezogen auf das Gesamtgewicht des Gemisches bevorzugt sind;
Stabilisatoren, wie Antioxidanzien, Lichtstabilisatoren, Hydroperoxid-Vernichter, Radikalfänger, Stabilisatoren gegen mikrobiellen Befall;
Weichmacher, insbesondere die unten beschriebenen.

Ferner können Netz-, Konservierungs-, Spreng-, Adsorptions- und Formentrennmittel sowie Tenside, insbesondere anionische und nicht-ionische, wie z.B. Seifen und seifenähnliche Tenside, Alkylsulfate und -sulfonate, Salze von Gallensäuren, alkoxylierte Fettalkohole, alkoxylierte Alkylphenole, alkoxylierte Fettsäuren und Fettsäureglycerinester, die alkoxyliert sein können, und Solubilisierungsmittel, wie Cremophor (polyethoxyliertes Rizinusöl), Gelucire, Vitamin E-TPGS und Tween (ethoxylierte Sorbitanfettsäureester), zugesetzt werden (vgl. z.B. H. Sucker et al. Pharmazeutische Technologie, Thieme-Verlag, Stuttgart 1978). Da die erfindungsgemäßen Formulierungen beim Kontakt mit Wasser oder wäßrigen Lösungsmitteln Emulsionen ausbilden, kann ein Zusatz eines oberflächenaktiven Hilfsstoffs, insbesondere von Stoffen mit hohen HLB-Werten, vor allem von mehr als 8, 10 und insbesondere von über 15, gering, in der Regel in Mengen von weniger als 1 Gew.-%, gehalten werden. Vorteilhafterweise kann auf einen solchen Zusatz verzichtet werden.

Unter Hilfsstoffen im Sinne der Erfindung sind auch Substanzen zur Herstellung einer festen Lösung mit dem Wirkstoff zu verstehen. Diese Hilfsstoffe sind beispielsweise Pentaerythrit und Pentaerythrittetraacetat, Harnstoff, Phosphatide, wie Lecithin, sowie Zuckeralkohole, wie Xylit und Mannit, Zitronen- und Bernsteinsäure, Gallensäuren, Stearine und andere, wie z.B. bei J. L. Ford, Pharm. Acta Helv. 61, (1986), S. 69-88, angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, (1989), S. 98-101).

Zugesetzt werden können auch Hilfsstoffe, wie Geschmackskorrigentien und Geruchsmaskierungsmittel, insbesondere Süßstoffe und Aromen.

Eine diesbezügliche Ausgestaltung beruht auf fachmännischem Wissen, wie es beispielsweise in Fiedler, H.B., Lexikon der Hilfsstoffe für Pharmazie, Kosmetik, und angrenzende Gebiete, 4.Aufl., Aulendorf: ECV-Editio-Cantor-Verlag (1996), dargestellt ist.

Hilfsstoffe im erfindungsgemäßen Sinn sind auch dosierungsformspezifische, d.h. auf eine bestimmte Dosierungsform, insbesondere perorale und vor allem Tabletten und Kapseln ausgerichtete Vehikel, auch niedrigschmelzende bzw. flüssige Hilfsstoffe, wie Polyalkylenglykole mit niedrigem Molekulargewicht, insbesondere Polyethylenglykol und/oder Polypropylenglykol mit gewichtsmittleren Molekulargewichten von weniger als 1.000, Wasser oder geeignete wässrige Systeme.

Die Hilfsstoffkomponente erfindungsgemäßer fester Formulierungen enthält vorzugsweise wenigstens einen der zuvor beschriebenen Hilfsstoffe. Sie kann weitere dieser Hilfsstoffe und/oder andere. Hilfsstoffe enthalten.

Eine Ausführungsform der vorliegenden Erfindung sind Formulierungsgrundlagen mit Hilfsstoffkomponente. In diesem Fall kann der Anteil an weiteren Hilfsstoffen in erfindungsgemäßen Formulierungen bis zu 30 Gew.-%, vorzugsweise 1 bis 20 Gew.-% und insbesondere 6 bis 15 Gew.-% betragen.

Eine besondere Ausführungsform der vorliegenden Erfindung sind Formulierungen, die
i) wenigstens einen Wirkstoff, vorzugsweise einen pharmazeutischen Wirkstoff;
ii) wenigstens eine ungesättigte Fettsäure, die vorzugsweise ausgewählt ist unter Ölsäure, Linolsäure und/oder Linolensäure, oder entsprechenden Mono- oder Diglyceriden;
iii)wenigstens ein Bindemittel, das ausgewählt ist unter Polyvinylpyrrolidonen, Vinylpyrrolidon-Copolymerisaten insbesondere mit Vinylacetat, oder Cellulosederivaten, insbesondere Hydroxypropylcellulosen und Hydroxypropylmethylcellulosen; und
iv) gegebenenfalls weitere Hilfsstoffe, beispielsweise ein Fließmittel,
enthalten.

Eine weitere besondere Ausführungsform der vorliegenden Erfindung sind Formulierungen, die
i') wenigstens einen lipidartigen Wirkstoff, vorzugsweise ein Öl und insbesondere ein ätherisches Öl;
ii') gegebenenfalls ein oder mehrere weitere Lipide;
iii) wenigstens einen Zuckeralkohol; insbesondere Maltit und/oder Isomalt, und wenigstens ein Stärkeverzuckerungsprodukt, insbesondere Maltodextrin; und
iv) gegebenenfalls weitere Hilfsstoffe
enthalten. Insbesondere können derartige Formulierung auch weitere Bindemittel, wie die oben genannten modifizierten natürlichen und synthetischen Polymere, z.B. Polyvinylpyrrolidone, Vinylpyrrolidon-Copolymerisate insbesondere mit Vinylacetat, oder Cellulosederivate, insbesondere Hydroxypropylcellulosen, Hydroxypropylmethylcellulosen oder Methylcellulose, oder Polyethylenglykole enthalten.

Die erfindungsgemäßen Formulierungen enthalten vorzugsweise weniger als 5 Gew.-% und insbesondere weniger als 1 Gew.-% Wasser. Eine besondere Ausführungsform stellen im wesentlichen wasserfreie Formulierungen dar.

Die erfindungsgemäßen Formulierungen sind vorzugsweise von fester Konsistenz. Der Begriff "fest" besitzt hier die in einschlägigen Arzneibüchern in Zusammenhang mit Arzneizubereitungen zugeordnete Bedeutung. Des weiteren können erfindungsgemäße Formulierungen auch von halbfester oder flüssig-viskoser Konsistenz sein. Auch die Begriffe "halbfest" und "flüssig-viskos" besitzen im Rahmen der vorliegenden Erfindung die in einschlägigen Arzneibüchern, in Zusammenhang mit Arzneizubereitungen zugeordneten Bedeutungen. Beispielsweise können bei verhältnismäßig hohen Anteilen an Lipiden und vor allem niedrigschmelzenden Lipiden, erfindungsgemäße Formulierungen von halbfester Konsistenz sein. Eine halbfeste und gewünschtenfalls auch flüssig-viskose Konsistenz kann bekanntermaßen auch durch den Zusatz geeigneter Hilfstoffe, insbesondere niedrigschmelzender oder flüssiger Vehikel, erzielt werden.

Gegenstand der vorliegenden Erfindung ist daher auch die Verwendung einer erfindungsgemäßen Formulierung gegebenefalls unter Zusatz weiterer Hilfsstoffe als Dosierungsform zur Anwendung wenigstens eines Wirkstoffs im Life-Science-Bereich.

Demnach finden erfindungsgemäße Formulierungen vornehmlich Anwendung im Life-Science-Bereioh. Hierzu gehört insbesondere der pharmazeutische, sowohl human- als auch tiermedizinische Bereich. In diesem Sinne finden die Formulierungen Anwendung als oder in Arzneiformen, d.h. die erfindungsgemäßen Formulierungen weisen, erforderlichenfalls zusammen mit weiteren Hilfsstoffen, zweckmäßige, der pharmazeutischen Praxis entsprechende Formen auf. Analoges gilt für den kosmetischen Bereich und angrenzende Gebiete wie den Pflanzenschutz, Nahrüngsmittelbereich und ähnliches. Zusammenfassend wird hier von einer Anwendung als oder in Dosierungsformen gesprochen, wobei der Begriff "Dosierungsform" eine mit Blick auf die Anwendung geformte Formulierung meint.

So bezeichnet der Begriff "Arzneiform" eine beliebige Dosierungsform zur Verabreichung von Wirkstoffen an einen Organismus, vorzugsweise an Säuger, insbesondere Menschen, und auch Nutz- oder Haustiere.

Zu gängigen Dosierungsformen gehören insbesondere Arzneiformen, wie (in alphabetischer Reihenfolge), Emulsionen und Mikroemulsionen, Granulate, Kapseln, Pellets, Pulver, Suspensionen, Suppositorien, Tabletten, insbesondere überzogene Tabletten, sowie analoge Dosierungformen zur Anwendung in anderen Life-Science-Bereichen.

Emulsionen bzw. Mikroemulsionen können vom Öl-in-Wasser- oder Wasser-in-Öl-Typ sein und enthalten die erfindungsgemäßen Formulierungen als disperse bzw. dispergierende Phase. Zur Stabilisierung können diese Emulsionen bzw. Mikroemulsionen Emulgatoren, die bekanntermaßen zu diesem Zweck verwendet werden, enthalten. Ein Vorteil erfindungsgemäßer Formulierungen ist es allerdings, daß in der Regel nur geringe Mengen an Emulgator zugesetzt werden und gemäß einer besonderen Ausführungsform der vorliegenden Erfindung auf einen Zusatz von Emulgatoren, insbesondere von O/W-Emulgatoren mit HLB-Werten von über 10 und insbesondere von über 15 verzichtet werden kann.

Granulate bestehen aus festen Körnern erfindungsgemäßer Formulierungen, wobei jedes Korn ein Agglomerat aus Pulverpartikeln darstellt. Als Arzneiform sind Granulate vorzugsweise zur oralen Anwendung bestimmt. Dem Anwender können Einzeldosiszubereitungen, beispielsweise in einem Beutelchen (Sachet), einem Papiersack oder einem Fläschchen abgepackte Granulate, oder Mehrdosenzubereitungen, die eine entsprechende Abmessung erfordern, angeboten werden. Vielfach stellen derartige Granulate aber nicht die eigentliche Arzneiform dar, sondern sie sind Zwischenprodukte bei der Herrichtung bestimmter Arzneiformen, beispielsweise um als Tablettengranulat zu Tabletten verpreßt, als Kapselgranulat in Hartgelatinekapseln abgefüllt, oder als Trink- oder Trockensaftgranulate vor der Einnahme zunächst in Wasser gegeben zu werden.

Als Kapseln sind die erfindungsgemäßen Formulierungen in der Regel in einer harten, aus zwei Teilen zusammengesteckten oder einer weichen, einteiligen, geschlossenen Hülle von unterschiedlicher Form und Größe abgefüllt. Die Ein- oder Umhüllung bzw. Matrixeinbettung erfindungsgemäßer Formulierungen in geeignete Polymeren, also Mikrokapseln bzw. Mikrospherulen ist ebenfalls möglich. Hart- wie Weichkapseln bestehen überwiegend aus Gelatine, wobei letztere einen geeigneten Anteil an weichmachenden Substanzen, wie Glycerol oder Sorbitol, aufweisen. Hartgelatine-Kapseln dienen zur Aufnahme erfindungsgemäßer Formulierungen, die eine feste Konsistenz aufweisen, beispielsweise als Granulat, Pulver oder Pellets. Weichgelatine-Kapseln bieten sich vor allem bei Formulierungen mit halbfester Konsistenz und erwünschtenfalls auch flüssig-viskoser Konsistenz an.

Pellets sind Granulate erfindungsgemäßer Formulierungen im Korngrößenbereich von ca. 0,5 bis 2 mm Durchmesser. Bevorzugt sind Pellets mit einer engen Korngrößenverteilung, vorzugsweise von 0,8 bis 1,2 mm, sowie im wesentlichen runder Gestalt.

In halbfesten Zubereitungen sind erfindungsgemäße Formulierungen in einem geeigneten Vehikel aufgenommen. Entsprechende Grundlagen sind dem Galeniker bekannt.

Suppositorien sind feste Zubereitungen zur rektalen, vaginalen oder urethralen Applikation. Um dem Verabreichungsweg gerecht zu werden, sind erfindungsgemäße Formulierungen in diesen Arzneiformen in der Regel in geeigneten Vehikeln aufgenommen, beispielsweise in bei Körpertemperatur schmelzenden Fetten wie Hartfett, Macrogole, d.h. Polyethylenglykolen mit Molekulargewichten von 1.000 bis 3.000 in verschiedenen Anteilen, Glycerolgelatine und ähnlichem.

Tabletten sind feste Zubereitungen vor allem zur oralen Anwendung. Oral besitzt im Rahmen der vorliegenden Erfindung insbesondere die Bedeutung des Begriffs "Peroral", d.h. Tabletten zur Resorption bzw. Wirkung des Wirkstoffs im Gastrointestinaltrakt. Besondere Ausführungsarten sind überzogene Tabletten, Schichttabletten, Manteltabletten, Tabletten mit modifizierter Wirkstoff-freisetzung, Matrixtabletten, Brausetabletten, Kautabletten oder Pillen. In der Regel enthalten die erfindungsgemäßen Formulierungen wenigstens einen Teil erforderlicher Tablettenhilfsstoffe, wie Bindemittel, Füllstoffe, Gleit- und Schmiermittel, bzw. Sprengmittel. Erforderlichenfalls können Tabletten erfindungsgemäßer Formulierungen auch weitere geeignete Hilfsstoffe umfassen. Zu nennen sind in diesem Zusammenhang insbesondere Hilfsstoffe, welche die Tablettierung unterstützen, beispielsweise Schmier- und Gleitmittel, z.B. die oben genannten, wobei vor allem zwecks erleichterter Komprimierung Magnesiumstearat bevorzugt ist.

Überzogene Tabletten weisen darüber hinaus geeignete Überzugsmaterialien, beispielsweise Filmlacke oder Dragierhilfsmittel, vor allem die unten genannten auf. Zu den überzogenen Tabletten gehören insbesondere Dragees und Filmtabletten.

Pulver sind fein disperse Feststoffe erfindungsgemäßer Formulierungen mit Korngrößen von in der Regel weniger als 1 mm. Obige Ausführungen zu Granulaten gelten entsprechend.

Erfindungsgemäß bevorzugt sind Kapseln, die mit zerkleinertem Granulat, Pulver oder Pellets erfindungsgemäßer Formulierungen befüllt sind, Trink- und Trockensaftgranulate aus erfindungsgemäßen Formulierungen und einem Zusatz an Geschmackskorrigentien, sowie insbesondere Tabletten.

In der Regel sind die erfindungsgemäßen Arzneiformen in geeigneter Form verpackt. Durchdrückpackungen aus Kunststoff und/oder Metall für feste Arzneiformen kommen häufig zur Anwendung.

Gegenstand der vorliegenden Erfindung ist auch ein Verfahren zur Herstellung einer erfindungsgemäßen Formulierung durch Vermischen der Komponenten i), ii), iii) und gegebenenfalls iv) unter Ausbildung eines plastischen Gemisches. Somit sind für die Bildung des plastischen Gemisches wenigstens zwei Maßnahmen erforderlich, einerseits das Vermischen der gemischbildenden Komponenten, andererseits deren Plastifizieren, d.h. deren Überführung in den plastischen Zustand. Diese Maßnahmen können für einzelne oder mehrere Komponenten oder Komponententeile nacheinander, ineinandergreifend, alternierend oder in sonstiger Weise erfolgen. Demnach ist es grundsätzlich möglich, während eines Mischvorganges gleichzeitig in den plastischen Zustand zu überführen, oder zunächst zu vermischen und dann das Gemisch in den plastischen Zustand zu überführen. Im Laufe eines Verfahrens können mehrere plastische Gemische unterschiedlicher Zusammensetzung gebildet werden, die miteinander und/oder mit weiteren Komponenten oder Komponententeilen vermischt werden. Beispielsweise kann eine Vormischung aus einem Teil der Komponenten unter Ausbildung eines plastischen Gemisches granuliert werden, und das Granulat kann dann unter Zusatz weiterer Komponenten in ein weiteres plastisches Gemisch überführt werden, dessen Zusammensetzung derjenigen der Formulierung entsprechen kann. Es können auch sämtliche Komponenten zunächst zusammengegeben und dann entweder gleichzeitig mit dem Vermischen in den plastischen Zustand, oder zunächst vermischt und anschließend in den plastischen Zustand überführt werden.

Die Bildung eines plastischen Gemisches kann durch Aufschmelzen, oder - unter zusätzlichem Eintrag mechanischer Energie, z.B. durch Kneten, Vermischen oder Homogenisieren - auch unterhalb der Schmelztemperatur des Gemisches erfolgen. Vorzugsweise bildet man das plastische Gemisch bei Temperaturen unterhalb von 220°C. In der Regel erfolgt die Bildung des plastischen Gemischs nicht durch Anteigen oder partielles Lösen einer oder mehrerer Komponenten mit Flüssigkeiten oder Lösungsmitteln, sondern hauptsächlich oder ausschließlich durch thermische oder thermisch-mechanische Einwirkung auf die Komponente(n), d.h. durch thermisches Plastifizieren. Bevorzugt erfolgt die Bildung des plastischen Gemischs durch Extrusion, besonders bevorzugt durch Schmelzextrusion. Die Verfahrensschritte des Plastifizierens können auf an sich bekannte Art und Weise durchgeführt werden, beispielsweise wie in der EP-A-0 240 904, EP-A-0 337 256, EP-A-0358 108, WO 97/15290 und WO 97/15291 beschrieben. Auf den Inhalt dieser Publikationen und insbesondere die darin enthaltenen Ausführungen zur Schmelzextrusion wird hiermit Bezug genommen.

Die Bindemittelkomponente sollte sich in der Gesamtmischung aller Komponenten im Bereich von 30 bis 200°C, vorzugsweise 40 bis 170°C in einen plastischen Zustand überführen lassen. Die Glasübergangstemperatur der Mischung sollte daher unter 220°C, vorzugsweise unter 180°C liegen. Erforderlichenfalls wird sie durch übliche, pharmakologisch akzeptable weichmachende Hilfsstoffe herabgesetzt.

Beispiele für derartige Weichmacher sind:
organische, vorzugsweise schwerflüchtige Verbindungen, wie z.B. C₇-C₃₀-Alkanole, Ethylenglykol, Propylenglykol, Glycerin, Trimethylolpropan, Triethylenglykol, Butandiole, Pentanole, wie Pentaerythrit und Hexanole, Polyalkylenglykole, vorzugsweise mit einem Molekulargewicht von 200 bis 1000, wie z.B. Polyethylenglykole, Polypropylenglykole und Polyethylenpropylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt soweit vorhanden im Allgemeinen 0,5 bis 30, vorzugsweise 0,5 bis 10 Gew.-%, bezogen auf das Gesamtgewicht von Polymer und Weichmacher.

Die Menge an Weichmacher beträgt vorteilhafterweise höchstens 30 Gew.-%, bezogen auf das Gesamtgewicht von Bindemittel und Weichmacher, damit - im Bereich fester Formen - lagerstabile Formulierungen und Arzneiformen gebildet werden, die keinen kalten Fluss zeigen. In der Regel ist der Zusatz eines Weichmachers zwecks Plastifizierung nicht erforderlich, da die erfindungsgemäß vorhandene Lipidkomponente weichmachende Eigenschaften besitzt.

Das erfindungsgemäße Verfahren kann vorteilhaft bei Temperaturen unterhalb von 200°C und bevorzugt unterhalb von 170°C, aber oberhalb von Raumtemperatur (25°C), vorzugsweise oberhalb von 40°C, durchgeführt werden. Insbesondere führt man das Verfahren in einem Temperaturintervall durch, das sich 40°C, bevorzugt 30°C und besonders bevorzugt 20°C von der Erweichungstemperatur des Gemischs der Komponenten nach oben oder unten erstreckt.

In bestimmten Fällen kann es vorteilhaft sein, Komponenten oder Teile von Komponenten als Lösung oder Suspension in einem Lösungsmittel zuzugeben. Zweckmäßig sind insbesondere niedermolekulare flüchtige Lösungsmittel, z.B. Wasser, C₁-C₆-Monoalkohole und deren Ether, Ester von C₁-C₆-Monoalkanolen mit C₁-C₆-Carbonsäuren, Alkane. Ein weiteres brauchbares Lösungsmittel ist flüssiges CO₂. Wasserlösliche Wirkstoffe können als wässrige Lösung eingesetzt werden oder vorzugsweise in eine wässrige Lösung oder Dispersion der Bindemittelkomponente oder eines Teils davon aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert. Die erfindungsgemäß einzusetzenden Komponenten können geringe Mengen Lösungsmittel enthalten, z.B. aufgrund von Hygroskopie, Lösungsmitteleinschlüssen oder Kristallwasser. Der Gesamtlösungsmittelgehalt des plastischen Gemisches liegt vorzugsweise unter 15%, insbesondere unter 10% und besonders bevorzugt unter 5%. Vorzugsweise erfolgt die Bildung des plastischen Gemisches ohne Zusatz eines Lösungsmittels, d.h. insbesondere durch lösungsmittelfreie Schmelzextrusion.

Die Komponenten, d.h. Wirkstoff, Lipid und Bindemittel sowie gegebenenfalls weitere Hilfsstoffe, können zunächst vermischt und dann in den plastischen Zustand überführt und homogenisiert werden. Insbesondere bei Verwendung empfindlicher Wirkstoffe hat es sich aber als vorteilhaft erwiesen, zunächst wenigstens einen Teil der Bindemittelkomponente und wenigstens einen Teil der Lipidkomponente, gegebenenfalls zusammen mit weiteren Hilfsstoffen, in den plastischen Zustand zu überführen. Hierzu können die Apparaturen, wie Rührkessel, Rührwerke, Feststoffmischer etc., im Wechsel betrieben werden. Anschließend können empfindliche Wirkstoffe eingemischt (homogenisiert) werden, vorzugsweise in "Intensivmischern" in plastischer Phase bei sehr kleinen Verweilzeiten. Der (die) Wirkstoff(e) kann (können) als solche, d.h. in fester, halbfester oder flüssiger Form oder als Lösung, Suspension oder Dispersion eingesetzt werden.

In bestimmten Ausführungsformen des erfindungsgemäßen Verfahrens kann es vorteilhaft sein, zunächst ein plastisches Gemisch aus Bindemittel und Wirkstoff und gegebenenfalls Hilfsstoffen zu bilden und zu diesem Gemisch das Lipid zu geben. Diese Vorgehensweise kann insbesondere dann vorteilhaft sein, wenn der Wirkstoff weichmacherähnliche Eigenschaften besitzt und die dadurch erreichbare Erniedrigung der Gesamtverfahrenstemperatur wünschenswert ist.

In bestimmten Ausführungsformen des erfindungsgemäßen Verfahrens kann es vorteilhaft sein, zunächst Wirkstoff und Lipid zu vermischen und dann zu plastifiziertem Bindemittel zu geben. Diese Vorgehensweise kann insbesondere dann vorteilhaft sein, wenn Wirkstoff und/oder Lipid thermisch labil sind.

Das Plastifizieren, Aufschmelzen und/oder Vermischen erfolgt in einer für diesen Zweck üblichen Vorrichtung. Besonders geeignet sind Extruder oder beheizbare Behälter mit Rührwerk, z.B. Kneter, (wie der unten noch erwähnten Art).

Als Mischapparat sind auch solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z.B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z.B. Dispax der Firma IKA).

Bei empfindlichen Wirkstoffen erfolgt vorzugsweise zunächst das Überführen der Bindemittelkomponente und der Lipidkomponente in den plastischen Zustand, z.B. in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Die Verfahrensschritte Vermischen und Plastifizieren, also insbesondere das Aufschmelzen, können in derselben Apparatur oder in zwei oder mehreren getrennt voneinander arbeitenden Vorrichtungen ausgeführt werden. Die Zubereitung einer Vormischung kann in einer der oben beschriebenen üblichen insbesondere zur Granulierung verwendeten Mischvorrichtungen durchgeführt werden. Eine solche Vormischung kann dann direkt, z.B. in einen Extruder, eingespeist und anschließend gegebenenfalls unter Zusatz weiterer Komponenten extrudiert werden.

Das erfindungsgemäße Verfahren erlaubt es, als Extruder Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Brauchbar sind z.B. Extruder der ZSK-Baureihe von Werner u. Pfleiderer.

Das Beschicken der Mischvorrichtung erfolgt je nach deren Konzeption kontinuierlich oder diskontinuierlich in üblicher Weise. Pulverförmige Komponenten können im freien Zulauf, z.B. über eine Differentialdosierwaage eingeführt werden. Plastische Massen können direkt aus einem Extruder eingespeist oder über eine Zahnradpumpe, die insbesondere bei hohen Viskositäten und hohen Drücken von Vorteil ist, zugespeist werden. Flüssige Medien können über ein geeignetes Pumpenaggregat zudosiert werden.

Die Lipidkomponente kann - wie vorstehend beschrieben - kontinuierlich oder diskontinuierlich in die Formulierung eingearbeitet werden. So kann wenigstens ein Teil der Lipidkomponente zunächst auf wenigstens einen Teil der Bindemittelkomponente (Matrix) geträgert werden, und anschließend als Vormischung unter Ausbildung eines plastischen Gemisches und eventueller Zugabe weiterer Bestandteile, vorzugsweise durch Extrusion erfindungsgemäß formuliert werden. Bevorzugt ist die kontinuierliche Zugabe wenigstens eines Teils der Lipidkomponente zu einem plastischen Gemisch. Dies ist insbesondere dann bevorzugt, wenn die erfindungsgemäß zu verwendenden Lipide in halbfester oder flüssiger Form verarbeitet werden können. Demnach sind auch aus verfahrenstechnischen Gründen die zuvor beschriebenen Lipide bevorzugt, die relativ geringe Schmelzpunkte aufweisen, von denen wiederum diejenigen bevorzugt sind, die bei Raumtemperatur, d.h. etwa 20 bis 30°C, von halbfester (wachsartig) und vorteilhafterweise von flüssiger Konsistenz (Öle) sind. Es ist bevorzugt, diese direkt in die Mischvorrichtung, insbesondere einen Extruder, zuzudosieren. Dies kann einen separat zu führenden Granuliernngsschritt ersparen. Besonders vorteilhaft ist die kontinuierliche Einbettung der lipidartigen Wirkstoffe, also insbesondere der etherischen Öle, in die entsprechende Bindemittelmatrix, vorzugsweise in einem Extruder, wobei das Öl kontinuierlich in einen das Bindemittelgemisch führenden Extruder eingespeist wird und die resultierende Formulierung also plastisches Gemisch extrudiert wird.

Das durch Vermischen und Überführen der Bindemittelkomponente, der Wirkstoffkomponente, der Lipidkomponente und gegebenenfalls weiterer Hilfsstoffe, in den plastischen Zustand erhaltene Gemisch ist teigig, zähflüssig oder dünnflüssig (thermoplastisch) und daher auch extrudierbar. Die Glasübergangstemperatur des Gemisches liegt vorteilhafterweise unter der Zersetzungstemperatur jeder in dem Gemisch enthaltenen Komponenten.

Die erfindungsgemäße Formulierung als plastisches Gemisch - gegebenenfalls nach dem Abkühlen oder Erstarren -, insbesondere als Extrudat, eignet sich für alle gängigen Verfahren zur Herrichtung gängiger Dosierungsformen.

Gegenstand der vorliegenden Erfindung ist auch ein Verfahren zur Herstellung von Dosierungsformen erfindungsgemäßer Formulierungen, wobei man die Formulierung nach obigem Verfahren herstellen kann und die Formulierung gegebenenfalls unter Zugabe weiterer Hilfsstoffe in die gewünschte Dosierungsform bringt. Dazu kann man formgebende Verfahrensmaßnahmen anwenden, wie das Ausformen des plastischen Gemisches, insbesondere durch Extrusion beziehungsweise Schmelzextrusion, und das Ausformen des plastischen Gemisches, insbesondere des Extrudats - gegebenenfalls nach dem Abkühlen oder Erstarren - z.B. durch Granulieren, Mahlen, Pressen, Formgießen, Spritzgießen, Tablettieren unter Druck, Tablettieren unter Druck und Wärme. Man kann eine Formulierung auch dadurch in eine gewünschte Dosierungsformen bringen, daß man sie in geeignete Vehikel einbringt. So können auch an sich feste Formulierungen zu halbfesten oder flüssigen Formulierungen durch den Zusatz geeigneter Vehikel verarbeitet werden.

So lassen sich eine Vielzahl von insbesondere festen Dosierungsformen herrichten. Beispielsweise kann man durch Mahlen oder Zerhacken des erstarrten oder zumindest teilweise erstarrten plastischen Gemischs Pulver oder Granulate herstellen, die entweder direkt zur Anwendung eingesetzt oder gegebenenfalls unter Zugabe üblicher Hilfsstoffe zu obigen Dosierungsformen, vor allem zu Tabletten, weiterverarbeitet werden können.

Vorzugsweise werden Dosierungsformen vor dem Erstarren des plastischen Gemischs geformt, die, gegebenenfalls nach Beschichten, Coaten, Dragieren oder mit einem Film überzogen, in einsetzbarer Form anfallen.

Die Formung zur Dosierungsform vor dem Erstarren kann in Abhängigkeit von der Viskosität des plastischen Gemischs auf vielfältige Weise erfolgen, z.B. durch Formgießen, Spritzgießen, Pressen, Quetschen oder Kalandrieren. Dazu wird das vorstehend beschriebene plastische Gemisch im erfindungsgemäßen Verfahren einem oder mehreren Formungsschritten zugeführt. Das Zuführen kann durch Pressen, Pumpen, z.B. mit Zahnradpumpen, oder vorzugsweise mit einem Extruder erfolgen.

Besonders bevorzugt wird das plastische Gemisch in einem oder mehreren, bevorzugt einem, Extruder gebildet und mit diesem oder einem nachgeschalteten Extruder den Formungsschritten zugeführt. In vielen Fällen hat es sich als vorteilhaft herausgestellt, schräg abwärts zu extrudieren und/oder gegebenenfalls eine Führungsrinne zum Transport des Extrudats vorzusehen, um einen sicheren Transport zu gewährleisten und ein Abreißen des extrudierten Stranges zu vermeiden.

In Abhängigkeit von der Anzahl und Verträglichkeit der einzusetzenden Wirkstoffe können vorteilhaft auch mehrschichtige Extrudate, z.B. Coextrudate, wie in der WO 96/19963 beschrieben, mitdem erfindungsgemäßen Verfahren verarbeitet werden.

Mehrschichtige feste Dosierungs- und insbesondere Arzneiformen, können vor allem durch Koextrusion hergestellt werden, wobei mehrere Gemische aus einzelnen oder mehreren der oben beschriebenen Komponenten bei der Extrusion so in einem Werkzeug zusammengeführt werden, dass sich der gewünschte Schichtaufbau ergibt. Vorzugsweise verwendet man verschiedene Bindemittel für verschiedene Schichten.

Mehrschichtige Dosierungs- und insbesondere Arzneiformen umfassen vorzugsweise zwei oder drei Schichten. Sie können in offener oder geschlossener Form vorliegen, insbesondere als offene oder geschlossene Mehrschichttabletten.

Erfolgt das Ausformen durch Koextrusion, so werden die Gemische aus den einzelnen Extrudern oder anderen Aggregaten in ein gemeinsames Koextrusionswerkzeug geführt und ausgetragen. Die Form der Koextrusionswerkzeuge richtet sich nach der gewünschten Arzneiform. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von der zur Anwendung kommenden Formulierungsgrundlage, und insbesondere der Bindemittelkomponente und der gewünschten Form.

Der erste Formungsschritt erfolgt vorteilhaft beim Austrag des Extrudats aus dem Extruder durch geeignet geformte Düsen, Blenden oder sonstige Austrittsöffnungen, z.B. durch eine Lochblende, eine Runddüse oder eine Breitschlitzdüse. In der Regel wird so kontinuierlich ein Strangextrudat mit vorzugsweise konstantem Querschnitt, z.B. in Form eines Bandes oder eines Stranges, vorzugsweise mit rundem, ovalem, abgerundetem oder flachem und breitem Querschnitt, erhalten.

Geeignete nachgeschaltete Formungsschritte für Extrudate sind z.B. der Kaltabschlag, d.h. das Schneiden beziehungsweise Zerhakken des Stranges nach zumindest teilweisem Erstarren, der Heißabschlag, d.h. das Zerschneiden beziehungsweise Zerhacken des Stranges in noch plastischer Form oder das Abquetschen des noch plastischen Strangs in einer Quetschvorrichtung. Mit Heiß- oder Kaltabschlag lassen sich z.B. Granulate (Heiß- oder Kaltgranulierung) oder Pellets erhalten. Die Heißgranulierung führt in der Regel zu Dosierungsformen (Pellets) mit einem Durchmesser von 0,5 bis 3 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene, mehrschichtige Dosierungsformen hergestellt werden, beispielsweise Oblongtabletten, Pastillen und Pellets. Die Dosierungsformen können in einem nachgeschalteten Verfahrensschritt nach üblichen Methoden mit einem Coating versehen werden. Geeignete Materialien für Filmüberzüge sind die als polymere Bindemittel genannten Polymere, insbesondere Polyacrylate, wie die Eudragit®-Typen, Celluloseester, wie die Hydroxypropylcellulosephthalate, sowie Celluloseether, wie Ethylcellulose, Hydroxypropylmethylcellulose oder Hydroxypropylcellulose und Gelatine. Auch weitere Formungsschritte können sich anschließen, wie z.B. die Arrondierung oder Verrundung der durch den Heiß- oder Kaltabschlag erhaltenen Pellets mittels Arrondiervorrichtungen, wie in der DE-A-196 29 753 beschrieben.

Besonders bevorzugt werden alle Formungsschritte am noch plastischen Gemisch bzw. noch plastischen Extrudat durchgeführt. Neben dem Heißabschlag, gegebenenfalls mit nachfolgendem Arrondieren, eignet sich insbesondere ein Verfahren, bei dem man das plastische Gemisch in einem Formkalander zur Dosierungsform formt. Dazu wird ein noch plastisches Gemisch oder ein noch plastisches Extrudat einem geeigneten Formkalander zugeführt. Geeignete Formkalander weisen zur Formung in der Regel Formwalzen und/oder Bänder auf, wobei mindestens eine der Formwalzen und/oder mindestens eines der Bänder Vertiefungen zur Aufnahme und Formung des plastischen Gemischs aufweist. Vorzugsweise verwendet man einen Formkalander mit gegenläufig rotierenden Formwalzen, wobei mindestens eine der Formwalzen auf ihrer Oberfläche Vertiefungen zur Aufnahme und Formung des plastischen Gemischs aufweist. Geeignete Formkalander und Formwalzen enthaltende Vorrichtungen sind allgemein beispielsweise in der EP-A-0 240 904, EP-A-0 240 906 und WO 96/19962, geeignete Bänder und Bänder enthaltende Vorrichtungen allgemein beispielsweise in der EP-A-0 358 105 offenbart, auf die diesbezüglich hiermit Bezug genommen wird.

Die Formgebung des noch plastischen Gemischs oder noch plastischen Extrudats erfolgt vorzugsweise bei Schmelztemperaturen unterhalb von 220°C, besonders bevorzugt unterhalb von 180°C und ganz besonders bevorzugt unterhalb von 150°C, wie z.B. in den zur Bildung des plastischen Gemischs notwendigen Temperaturbereichen oder bei niedrigeren Temperaturen. Wenn die Formung bei niedrigeren Temperaturen erfolgt, erfolgt sie vorteilhaft 5 bis 70°C, bevorzugt 10 bis 50°C und besonders bevorzugt 15 bis 40°C unterhalb der höchsten bei der Bildung des plastischen Gemischs erreichten Temperatur, vorzugsweise jedoch oberhalb der Erstarrungstemperatur des plastischen Gemischs.

Die erfindungsgemäße Herstellung der Formulierungen und Zubereitung der Dosierungsformen kann ganz oder teilweise unter sterilen Arbeitsbedingungen durchgeführt werden, z.B. in Reinräumen und unter Verwendung sterilisierter Geräte, wie z.B. Waagen, Mischern, Extrudern und Formungsmaschinen, wie Kalandern, Quetschvorrichtungen und Zerhackern. Die Einsatzstoffe können entweder in sterilisierter Form, gegebenenfalls unter Zugabe geeigneter antibakterieller und/oder antiviraler Hilfsstoffe, in das Verfahren eingebracht werden und/oder die Verfahrensbedingungen, insbesondere die Temperatur, so gewählt werden, dass sterile Formulierungen bzw. Arzneiformen erhalten werden. Die erhaltenen sterilen Dosierungsformen können anschließend unter ebenfalls sterilen Bedingungen direkt verpackt werden, z.B. durch Verblistern oder Einschweißen. Die Formgebung und das Verpacken kann auch gleichzeitig durchgeführt werden, insbesondere wenn die Formgebung des plastischen Gemischs durch Kalandrieren mittels Formwalzen durchgeführt wird. Dazu bringt man zusätzlich zu dem plastischen Gemisch als Folien vorliegende Materialien jeweils zwischen Schmelze und Formwalze, wodurch gleichzeitig mit der Formung des plastischen Gemischs zu Dosierungsformen eine Umhüllung und/oder eine Verpackung der Dosierungsform erreicht werden kann, wie in der WO-96/19963 beschrieben, auf die diesbezüglich hiermit Bezug genommen wird.

Ein weiterer Gegenstand der vorliegenden Erfindung ist die Verwendung einer erfindungsgemäßen Formulierungsgrundlage bei der Anwendung wenigstens eines Wirkstoffs im Life-Science-Bereich, also insbesondere im Pharma-, Kosmetik-, Pflanzenschutz-, Nahrungsmittel- sowie Wasch-, Reinigungs- und Hygienebereich.

Zweck dieser Verwendung ist es insbesondere, die Wirkung der Wirkstoffkomponente zu verbessern. So beinhaltet diese Verwendung insbesondere ein Verfahren zur Verbesserung der Wirkung der Wirkstoffkomponente bei der Anwendung wenigstens eines Wirkstoffs im Life-Science-Bereich, wobei man eine erfindungsgemäße Formulierungsgrundlage vewendet. Dabei bringt man wenigstens einen Wirkstoff in diese Formulierungsgrundlage ein, vorzugsweise mit einem der vorstehend geschilderten Verfahren. Insbesondere dient die Bindemittelmatrix der Formulierungsgrundlage zur Aufnahme wenigstens eines Lipids bei der Herstellung einer erfindungsgemäßen festen Formulierung zur Verbesserung der Wirkung der Wirkstoffkomponente.

Die Bindemittelmatrix wird von der zuvor beschriebenen Bindemittelkomponente oder zumindest eines Teils davon gebildet. In diese Bindemittelmatrix wird wenigstens ein Lipid, das Bestandteil der zuvor beschriebenen Lipidkomponente und/oder der Wirkstoffkomponente ist, aufgenommen. Insbesondere bevorzugt ist es, dass die Aufnahme zu einer im Wesentlichen molekulardispersen Verteilung von Lipid in der Bindemittelmatrix führt. Eine homogene Verteilung von Lipid in der Matrix ist von Vorteil, insbesondere im Hinblick auf wirkstoffördernde Eigenschaften des Lipids. Diese Vorteile können auch erzielt werden, ohne den Wirkstoff molekulardispers zu verteilen. Lipide, die zur Verbesserung der pharmakologischen Wirkung eines Wirkstoffs, dienen können, sind dem Fachmann u.a. als Resorptionsförderer bekannt. Unter diesen kann er beispielsweise wenigstens einen Teil der Lipidkomponente auswählen. Ergänzend wird auf die Ausführungen oben in Zusammenhang mit der Beschreibung der Lipidkomponente Bezug genommen.

Die erfindungsgemäße Verwendung ist insbesondere immer dann von Vorteil, wenn Wirkstoffe auf eine Art und Weise angewendet werden sollen, bei der durch gleichzeitige Gabe von Lipiden ein wirkstoffördernder Effekt auftreten kann. Dies betrifft im Bereich der Pharmazie vor allem Verabreichungswege, die den Gastrointestinaltrakt mit einbeziehen, also insbesondere die enterale, vor allem rektale und vorzugsweise orale Verabfolgung. Ganz besonders von Vorteil ist die erfindungsgemäße Anwendung dann, wenn ein zu verabreichender pharmazeutischer Wirkstoff ohne geeignete Maßnahmen, wie dem Zusatz wenigstens eines Lipids, auf diesem Wege nur unzureichend angewendet werden kann.

Die Erfindung betrifft auch die Verwendung einer erfindungsgemäßen Formulierung gegebenefalls unter Zusatz weiterer Hilfsstoffe als Dosierungsform im Life-Science-Bereich.

Zu den Dosierungsformen gehören insbesondere die zuvor genannten Arzneiformen. Entsprechende Dosierungsformen für die kosmetische Anwendung, zur Pflanzenbehandlung, für die Ernährungstechnologie, Lebensmittel- und Futtertechnologie eingeschlossen, und weitere angrenzende Gebiete können unter Berücksichtigung zweckmäßiger anwendungsspezifischer Ausgestaltungen hergerichtet werden. Die erfindungsgemäße Verwendung richtet sich insbesondere auf die human- und tiermedizinische Behandlung, die kosmetische Behandlung, den Pflanzenschutz, die Ergänzung von Lebens- und Futtermitteln mit Wirkstoffen, und die Ausrüstung von Wasch-, Reinigungs- und Hygieneprodukten mit Wirkstoffen.

Eine besondere Verwendung betrifft den Zusatz erfindungsgemäßer Formulierungen in Hygieneprodukten, insbesondere Babywindeln. Im Rahmen dieser Verwendung finden insbesondere diejenigen Formulierungen Anwendung, die auf lipidartigen Wirkstoffen basieren. So können feste oder halbfeste Formulierungen mit Aromen oder anderen hydrophoben Wirkstoffen, z.B. antimikrobiell wirksamen Substanzen, insbesondere den zuvor genannten ätherischen Ölen, verwendet werden. Bei Kontakt mit Körperflüssigkeit bilden diese Formulierungen dann eine Emulsion aus, deren große Oberfläche insbesondere Aromen effektiv wirken läßt.

Erfindungsgemäße Arzneiformen werden dem zu behandelnden Individuum, vorzugsweise einem Säuger, insbesondere einem Menschen, und auch Nutz- oder Haustier, und damit eine wirksame Menge an Wirkstoff verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken bestimmte Anzeichen, Symptome und/oder Fehlfunktionen zu entwikkeln, und weitere Faktoren mit einbezieht. In der Regel werden die erfindungsgemäßen Arzneiformen einmal oder mehrmals am Tag zusammen oder im Wechsel mit anderen Präparaten derart verabreicht, daß einem zu behandelnden Individuum eine Tagedosis in , einer Menge zugeführt wird, die eine Therapie ermöglicht.

Die erfindungsgemäßen Formulierungen stellen selbstemulgierende Systeme dar. Bringt man die Formulierungen mit wäßrigen Medien in Kontakt, bilden sich Emulsionen. Dementsprechend betrifft die vorliegende Erfindung auch ein Verfahren zur Herstellung von Emulsionen. Diese Emulsionen sind in der Regel stabil, vor allem unter den Anwendungsbedingungen der erfindungsgemäßen Formulierungen. So bilden die Formulierungen in der Regel schon bei Temperaturen von weniger als 90°C stabile Emulsionen aus. Bevorzugte Temperaturbereiche für das Ausbilden stabiler Emulsionen liegen bei 5 bis 60°C und insbesondere bei 10 bis 40°C. Es handelt sich dabei vorteilhafterweise um feinteilige Emulsionen mit einem überwiegenden Anteil an Teilchen mit Durchmessern von weniger als 100 µm, vorzugsweise von weniger als 50 µm und insbesondere von weniger als 20 µm. Formulierungen, die beim Kontakt mit wäßrigem Medium Emulsionen ausbilden, bei denen mindestens 50% der Teilchendurchmesser in einem Bereich von 100 bis 20.000 nm, vorzugweise von 10 bis 5.000 nm und insbesondere von 300 bis 2.000 nm liegen, stellen bevorzugte Ausführungsformen dar.

Die Bildung der Emulsionen erfolgt unter Anwendungsbedingungen in der Regel spontan. Insbesondere ist ein nennenswerter Eintrag von mechanischer Energie, z.B. Rühr- und/oder Scherenergie, nicht erforderlich. So kann die Herstellung erfindungsgemäßer Formulierungen zunächst lösungsmittelfrei erfolgen. Die Ausbildung der Emulsion erfolgt dann je nach Anwendung beim Kontakt mit einem wäßrigen Medium, im Bereich von Arzneiformen vor der Verabreichung durch Zubereitung einer entsprechenden Dosierungsform oder nach Verabreichung bei Kontakt mit einer geeigneten Körperflüssigkeit.

Die Ausbildung feinteiliger Emulsionen wird vor allem durch den Zustand einer molekulardispersen Verteilung und insbesondere festen Lösung erfindungsgemäßer Formulierungen unterstützt.

So sind erfindungsgemäße Formulierungsgrundlagen insbesondere dann bevorzugt, wenn Lipidemulsionen bei der Anwendung von Wirkstoffen bevorzugt sind. Dies betrifft vor allem schwerlösliche Wirkstoffe; Wirkstoffe, die zwar gut löslich, aber bei enteraler Verabfolgung nur eine unzureichende Wirkung entfalten; und/oder Wirkstoffe, die lokale Reizungen und/oder andere unerwünschte Nebenwirkungen verursachen. Dies gilt für topische Applikationsformen, wie z.B. Lotionen, Cremes und Salben, genauso wie für parenterale Applikationsformen, wie z.B. Injektionslösungen, und orale Applikationsformen, z.B. Trinklösungen und feste Dosierungsformen, beispielsweise Tabletten und Kapseln.

Zur Ausbildung der Emulsionen kommt, vor allem die Verwendung der zuvor genannten Bindemittel zum tragen. Diese Bindemittel und insbesondere die polymeren Bindemittel können als Solubilisatoren dienen und somit im Hinblick auf die Emulgierung der Lipide die Funktion von Emulgatoren übernehmen. Ein weiterer Aspekt der vorliegenden Erfindung ist daher die Verwendung der Bindemittelkomponente zur Emulgierung der Lipidkomponente.

Die vorliegende Erfindung soll nun anhand der folgenden Beispiele veranschaulicht, nicht aber beschränkt werden.

### Beispiel 1:

Eine Mischung aus gleichen Gewichtsteilen Hydroxypropylcellulose (Klucel EF, Aqualon) und Ölsäure wurde bei 120°C in einem Meß-Kneter (Rheomix, Fa. Haake) zu einer homogenen, gummiartigen Schmelze verarbeitet. Nach dem Abkühlen lag eine transparente feste Masse vor, die sich in Wasser unter Bildung einer Emulsion löste.

### Beispiel 2:

Der Versuch erfolgte analog Beispiel 1, aber mit einer Mischung aus 60 Gew.-% Kollidon VA-64 (BASF) und 4.0 Gew.-% Ölsäure bei einer Temperatur von 100°C über 5 Minuten. Es wurde eine klare, niederviskose durchsichtige Schmelze erhalten, die nach Abkühlen auf Raumtemperatur (und auch nach über 12 Monaten Lagerung bei Raumtemperatur) klartransparent und noch plastisch verformbar war. Die erkaltete Schmelze löste sich leicht unter Bildung einer Emulsion in Wasser. Die Größe der Emulsionströpfchen dieser Zubereitung wurde mit Hilfe eines Mastersizer-Geräts (Fa. Malvern, UK) gemessen. 90 % der Partikel wiesen Größen von unter 35 µm auf, 50 % der Partikel waren kleiner als 2 µm.

### Beispiel 3:

Der Versuch erfolgte analog Beispiel 2, aber mit einer Mischung aus 72 Gew.-% Kollidon VA-64 (BASF) und 28 Gew.-% Ölsäure. Die erkaltete Schmelze war ebenfalls transparent, aber weniger leicht plastisch verformbar als in Beispiel 2. Die erkaltete Schmelze löste sich leicht in Wasser unter Bildung einer Emulsion. Die Größe der Emulsionströpfchen dieser Zubereitung wurde mit Hilfe eines Mastersizer-Geräts (Fa. Malvern, UK) gemessen. 90 % der Partikel wiesen Größen von unter 4 µm auf, 50 % der Partikel waren kleiner als 0,7 µm.

### Beispiel 4:

Der Versuch erfolgte wie in Beispiel 3, aber mit einer Mischung aus 64 Gew.-% Kollidon VA-64 (BASF), 16 Gew.-% Ölsäure und 20 Gew.-% Dextran bei 118°C. Es wurde eine weißliche Schmelze erhalten, die nach dem Abkühlen fest wurde, und die sich in 0,1 M HCl recht schnell unter Bildung einer Emulsion löste.

### Beispiel 5:

Der Versuch erfolgte wie in Beispiel 4, aber mit einer Mischung, bestehend aus 70 Gew.-% Hydroxypropylcellulose (Klucel EF, Aqualon), 10 Gew.-% Ölsäure und 20 Gew.-% Dextran bei 120°C. Es wurde eine weiße, nach dem Abkühlen feste Schmelze erhalten, die sich in Wasser unter Bildung einer Emulsion, aber langsamer als im Fall des Beispiels 4, auflöste.

### Beispiel 6:

Der Versuch erfolgte wie in Beispiel 5, aber mit einer Mischung bestehend aus 40 Gew.-% Hydroxypropylcellulose (Klucel EF, Aqualon), 40 Gew.-% Stearinsäure und 20 Gew.-% Dextran bei 120°C. Es wurde eine weiße Schmelze erhalten, die nach Abkühlen und Mahlen relativ leicht in Wasser unter Bildung einer Emulsion dispergierbar war.

### Beispiel 7:

Der Versuch erfolgte wie in Beispiel 1, aber mit einer Mischung bestehend aus 70 Gew.-% Hydroxypropylcellulose (Klucel EF, Aqualon) und 30 Gew.-% Stearinsäure bei 110°C. Es bildete sich eine klare, gummiartige Schmelze, die beim Abkühlen weiß erstarrte.

### Beispiel 8:

Zu 7 Gewichtsteilen Kollidon VA-64 (BASF) wurde 1 Gewichtsteil Ölsäure unter leichtem Kneten gegeben. Unter leichter Wärmeentwicklung entstand nach einigen Minuten Kneten ein homogenes Granulat, das mit 2 Gewichtsteilen des Wirkstoffs Esupron vermischt wurde. In diese Granulat-Mischung wurde dann noch 1 Gew.-% hochdisperses Kieselgel (Aerosil 200) gegeben und diese Mischung dann über eine Dosierwaage in einen Doppelschneckenextruder zudosiert (16 mm Schneckendurchmesser) und bei einer Temperatur von 110°C extrudiert. Es entstand eine klare Schmelze, die nach Abkühlen unter Bildung einer Emulsion in Wasser aufgelöst werden konnte.

### Beispiel 9:

Der Versuch wurde analog Beispiel 8 durchgeführt, aber mit 2 Gewichtsteilen Paracetamol, die mit einer zuvor granulierten Mischung aus 7 Gewichtsteilen Kollidon VA-64 (BASF) und 1 Gewichtsteil Ölsäure geknetet worden war. Zu der Gesamtmischung wurde unter Mischen Vor der Extrusion ebenfalls 1 Gewichts-% Aerosil 200 gegeben. Die Extrusion erfolgte bei einer Temperatur von 125°C, die erkaltete Schmelze löste sich unter Bildung einer feinteiligen Emulsion in Wasser.

### Beispiel 10:

Der Versuch erfolgte wie in Beispiel 8, aber mit 2 Gewichtsteilen Paracetamol und einer Granulat-Mischung aus 6,125 Gewichtsteilen Kollidon VA-64 (BASF), 0,875 Gewichtsteilen Ölsäure und 1 Gewichtsteil Stearylalkohol. Zu der Gesamtmischung wurde unter Mischen vor der Extrusion ebenfalls 1 Gewichts-% Aerosil 200 gegeben. Die Extrusion erfolgte bei einer Temperatur von 120°C, die erkaltete Schmelze löste sich unter Bildung einer feinteiligen Emulsion in Wasser.

Die nachfolgenden Beispiele 11 bis 13 veranschaulichen erfindungsgemäße Formulierungen mit lipidartigen Wirkstoffen, hier Orangenöl als ätherisches Öl. Die Gemische wurden in einem Doppelschneckenextruder ZSK30 der Firma Werner & Pfleiderer bei einem Durchsatz von 2,7 kg/Stunde verarbeitet. Die Formgebung des noch plastischen Extrudats erfolgte wie in der EP-A 240 906 beschrieben. Die seitliche Zudosierung erfolgte mit einer HPLC-Pumpe und einer Pumprate von 300 g/h im Schuß 2.

### Beispiel 11

2 Gew.-% eines PVP-Homopolymerisats mit einem K-Wert von 30 (Kollidon 30), 70 Gew.-% Isomalt, 18 Gew.-% Maltodextrin mit DE 15 (C-Pur 01915, Firma Cerestar) wurden in einem Doppelschneckenextruder vermischt. 10 Gew.-% Orangenöl wurden seitlich zudosiert und kontinuierlich in die Matrix eingearbeitet. Nach der Extrusion wurde das Gemisch mittels Heißabschlag zu Pellets von etwa 1 mm Größe konfektioniert. Die Temperatur der einzelnen Schüsse betrug 39°C, 57°C, 110°C, 89°C und 89°C, die der Düse 101°C.

### Beispiel 12

2 Gew.-% Hydroxypropylcellulose mit einem gewichtsmittleren Molekulargewicht von etwa 80.000 (Klucel EF), 70 Gew.-% Isomalt, 18 Gew.-% Maltodextrin mit DE 15 (C-Pur 01915, Firma Cerestar) wurden in einem Doppelschneckenextruder vermischt. 10 Gew.-%. Orangenöl wurden seitlich zudosiert und kontinuierlich in die Matrix eingearbeitet. Nach der Extrusion wurde das Gemisch mittels Heißabschlag zu Pellets von etwa 1 mm Größe konfektioniert. Die Temperatur der Schüsse betrug 61°C, 84°C, 120°C, 111°C und 100°C, die der Düse 111°C.

### Beispiel 13

2 Gew.-% eines PVP-Homopolymerisats mit einem K-Wert von 30 (Kollidon 30), 53 Gew.-% Isomalt, 35 Gew.-% Maltodextrin mit DE15 (C-Pur 01915, Firma Cerestar) wurden in einem Doppelschneckenextruder vermischt. 10 Gew.-% Orangenöl wurden seitlich zudosiert und kontinuierlich in die Matrix eingearbeitet. Nach der Extrusion wurde das Gemisch mittels Heißabschlag zu Pellets von etwa 1 mm Größe konfektioniert. Die Temperatur der Schüsse betrug 52°C, 64°C, 110°C, 92°C und 91°C, die der Düse 104°C.

## Patentansprüche

1. Selbstemulgierende Formulierung, enthaltend
i) 0,1 bis 50 Gew.-% einer aus wenigstens einem Wirkstoff gebildeten Wirkstoffkomponente, wobei der Anteil an Wirkstoffkristallen weniger als 5 % beträgt;
ii) 6 bis 60 Gew.-% einer aus wenigstens einem Lipid gebildeten Lipidkomponente, wobei die Lipidkomponente einen Schmelzpunkt von höchstens 50°C aufweist;
iii) 20 bis 93,9 Gew.-% einer aus einem oder mehreren unter Polyvinylpyrrolidonen, Vinylpyrrolidon-Vinylacetat-Copolymeren, Hydroxyalkylcellulosen, Hydroxyalkyl-Alkylcellulosen, Cellulosephthalaten, (Meth)acrylharzen ausgewählten Bindemittel(n) gebildeten Bindemittelkomponente; und
iv) 0 bis 30 Gew.-% weiterer Hilfsstoffe,
wobei der auf das Gesamtgewicht der Bindemittelkomponente bezogene Anteil der Lipidkomponente höchstens 40 Gew.-% beträgt.

2. Formulierung nach Anspruch 1, **dadurch gekennzeichnet, dass** die Formulierung im wesentlichen frei von Wirkstoffkristallen ist.

3. Formulierung nach Anspruch 1 oder 2, **dadurch gekennzeichnet, dass** der Wirkstoff in Wasser schwer löslich ist.

4. Formulierung nach Anspruch 3, **dadurch gekennzeichnet, dass** zum Lösen 1 Teils des in Wasser schwer löslichen Wirkstoffs wenigstens 100 Teile Wasser erforderlich sind.

5. Formulierung nach einem der Ansprüche 1 bis 4, **dadurch gekennzeichnet, dass** das Lipid ausgewählt ist unter Fettsäuren, Triglyceriden, Diglyceriden und Monoglyceriden.

6. Formulierung nach Anspruch 5, **dadurch gekennzeichnet, dass** das Lipid ausgewählt ist unter ungesättigten Fettsäuren sowie Mono-, Di- oder Trigyceriden ungesättigter Fettsäuren.

7. Formulierung nach Anspruch 6, **dadurch gekennzeichnet, dass** die ungesättigte Fettsäure ausgewählt ist unter Ölsäure, Linolsäure und Linolensäure.

8. Formulierung nach einem der Ansprüche 1 bis 7, **dadurch gekennzeichnet, dass** die Lipidkomponente einen HLB-Wert von höchstens 12 aufweist.

9. Formulierung nach einem der Ansprüche 1 bis 8, **dadurch gekennzeichnet, dass** die Lipidkomponente einen Schmelzpunkt von weniger als 30°C aufweist.

10. Formulierung nach einem der Ansprüche 1 bis 9, **dadurch gekennzeichnet, dass** die Lipidkomponente in molekulardisperser Form vorliegt.

11. Formulierung nach einem der Ansprüche 1 bis 10, **dadurch gekennzeichnet, dass** der Anteil der Lipidkomponente, bezogen auf die Bindemittelkomponente, höchstens 25 Gew.-% beträgt.

12. Formulierung nach einem der Ansprüche 1 bis 11, **dadurch gekennzeichnet, dass** das Bindemittel ausgewählt ist unter Polyvinylpyrrolidonen, Vinylpyrrolidon-Vinylacetat-Copolymerisaten, Hydroxypropylcellulosen und Hydroxypropylmethylcellulosen.

13. Formulierung nach einem der Ansprüche 1 bis 12, **dadurch gekennzeichnet, dass** der weitere Hilfsstoff ausgewählt ist unter Füllstoffen, Schmiermitteln, Gleitmitteln, Trennmitteln, Fließmitteln, Farbstoffen, Stabilisatoren, Weichmachern, Netzmitteln, Konservierungsmitteln, Sprengmitteln, Adsorptionsmitteln, Formentrennmitteln und Tensiden.

14. Formulierung nach einem der Ansprüche 1 bis 13, **dadurch gekennzeichnet, daß** sie durch Schmelzextrusion eines die Komponenten i), ii), iii) und gegebenenfalls iv) enthaltenden plastischen Gemisches erhältlich ist.

15. Selbstemulgierende Formulierung, enthaltend
i') eine aus wenigstens einem ätherischen Öl gebildete Wirkstoffkomponente;
ii') gegebenenfalls einen aus wenigstens einem weiteren Lipid gebildeten weiteren Lipidkomponentenanteil;
iii) eine wenigstens ein unter Polyvinylpyrrolidonen, Vinylpyrrolidon-Vinylacetat-Copolymeren, Hydroxyalkylcellulosen, Hydroxyalkyl-Alkylcellulosen, Cellulosephthalaten, (Meth)acrylharzen ausgewähltes Bindemittel umfassende Bindemittelkomponente; und
iv) gegebenenfalls weitere Hilfsstoffe,
wobei der auf das Gesamtgewicht Formulierung bezogene Anteil der Komponenten i') und ii') 6 bis 60 Gew.-% und der auf die Bindemittelkomponente bezogene Anteil der Komponenten i') und ii') höchstens 40 Gew.-% beträgt.

16. Formulierung nach Anspruch 15, **dadurch gekennzeichnet, dass** der auf das Gesamtgewicht der Formulierung bezogene Anteil der Komponenten i') und ii') 11 bis 40 Gew.-% beträgt.

17. Formulierung nach Anspruch 15 oder 16, **dadurch gekennzeichnet, dass** der auf das Gesamtgewicht der Formulierung bezogene Anteil ätherischen Öls 5 bis 20 Gew.-% beträgt.

18. Formulierung nach einem der Ansprüche 15 bis 17, **dadurch gekennzeichnet, dass** die Bindemittelkomponente auch wenigstens einen Zuckeralkohol und/oder wenigstens ein Stärkeverzuckerungsprodukt umfasst.

19. Formulierung nach Anspruch 18, **dadurch gekennzeichnet, dass** der Zuckeralkohol ausgewählt ist unter Maltit, Xylit und Isomalt.

20. Formulierung nach Anspruch 18, **dadurch gekennzeichnet, dass** das Stärkeverzuckerungsprodukt Maltodextrin ist.

21. Formulierung nach einem der Ansprüche 1 bis 20, **dadurch gekennzeichnet, dass** sie fest ist.

22. Verwendung einer Formulierung nach einem der Ansprüche 15 bis 21 in Hygieneprodukten.

## Claims

1. Self-emulsifying formulation comprising
i) 0.1 to 50% by weight of an active ingredient component formed from at least one active ingredient, where the content of active ingredient crystals is less than 5%;
ii) 6 to 60% by weight of a lipid component formed from at least one lipid, where the lipid component has a melting point not exceeding 50°C;
iii) 20 to 93.9% by weight of a binder component formed from one or more binder(s) selected from polyvinylpyrrolidones, vinylpyrrolidone/vinyl acetate copolymers, hydroxyalkylcelluloses, hydroxyalkylalkylcelluloses, cellulose phthalates, (meth)acrylic resins; and
iv) 0 to 30% by weight of other excipients,
where the content of the lipid component based on the total weight of the binder component is not more than 40% by weight.

2. Formulation according to Claim 1, **characterized in that** the formulation is essentially free of active ingredient crystals.

3. Formulation according to Claim 1 or 2, **characterized in that** the active ingredient is slightly soluble in water.

4. Formulation according to Claim 3, **characterized in that** at least 100 parts of water are necessary to dissolve 1 part of the active ingredient which is slightly soluble in water.

5. Formulation according to any of Claims 1 to 4, **characterized in that** the lipid is selected from fatty acids, triglycerides, diglycerides and monoglycerides.

6. Formulation according to Claim 5, **characterized in that** the lipid is selected from unsaturated fatty acids and mono-, di- or triglycerides of unsaturated fatty acids.

7. Formulation according to Claim 6, **characterized in that** the unsaturated fatty acid is selected from oleic acid, linoleic acid and linolenic acid.

8. Formulation according to any of Claims 1 to 7, **characterized in that** the lipid component has an HLB not exceeding 12.

9. Formulation according to any of Claims 1 to 8, **characterized in that** the lipid component has a melting point of less than 30°C.

10. Formulation according to any of Claims 1 to 9, **characterized in that** the lipid component is present in the form of a molecular dispersion.

11. Formulation according to any of Claims 1 to 10, **characterized in that** the content of the lipid component, based on the binder component, is not more than 25% by weight.

12. Formulation according to any of Claims 1 to 11, **characterized in that** the binder is selected from polyvinylpyrrolidones, vinylpyrrolidone/vinyl acetate copolymers, hydroxypropylcelluloses and hydroxypropylmethylcelluloses.

13. Formulation according to any of Claims 1 to 12, **characterized in that** the other excipient is selected from fillers, lubricants, glidants, separating agents, flow regulators, dyes, stabilizers, plasticizers, wetting agents, preservatives, disintegrants, adsorbents, mould release agents and surfactants.

14. Formulation according to any of Claims 1 to 13, **characterized in that** it is obtainable by melt extrusion of a plastic mixture comprising components i), ii), iii) and, where appropriate, iv).

15. Self-emulsifying formulation comprising
i') an active ingredient component formed from at least one essential oil;
ii') where appropriate another lipid component content formed from at least one other lipid;
iii) a binder component comprising at least one binder selected from polyvinylpyrrolidones, vinylpyrrolidone/vinyl acetate copolymers, hydroxyalkylcelluloses, hydroxyalkylalkylcelluloses, cellulose phthalates, (meth)acrylic resins; and
iv) where appropriate other excipients,
where the content of components i') and ii') is from 6 to 60% by weight based on the total weight of the formulation, and the content of components i') and ii') is not more than 40% by weight based on the binder component.

16. Formulation according to Claim 15, **characterized in that** the content of components i') and ii') is from 11 to 40% by weight based on the total weight of the formulation.

17. Formulation according to Claim 15 or 16, **characterized in that** the content of essential oil is from 5 to 20% by weight based on the total weight of the formulation.

18. Formulation according to any of Claims 15 to 17, **characterized in that** the binder component also comprises at least one sugar alcohol and/or at least one starch saccharification product.

19. Formulation according to Claim 18, **characterized in that** the sugar alcohol is selected from maltitol, xylitol and isomalt.

20. Formulation according to Claim 18, **characterized in that** the starch saccharification product is maltodextrin.

21. Formulation according to any of Claims 1 to 20, **characterized in that** it is solid.

22. Use of a formulation according to any of Claims 15 to 21 in hygiene products.

## Revendications

1. Formulation autoémulsifiante, contenant
i) 0,1 à 50 % en poids d'un composant de substance active formé d'au moins une substance active, la fraction de cristaux de substance active étant inférieure à 5 %,
ii) 6 à 60 % en poids d'un composant lipidique formé d'au moins un lipide, le composant lipidique présentant un point de fusion d'au maximum 50°C,
iii) 20 à 93,9 % en poids d'un composant liant formé d'un ou de plusieurs liants choisis parmi des polyvinylpyrrolidones, des copolymères de vinylpyrrolidone-acétate de vinyle, des hydroxyalkylcelluloses, des hydroxyalkyl-alkylcelluloses, des phtalates de cellulose, des résines (méth)acryliques, et
iv) 0 à 30 % en poids d'autres adjuvants,
la fraction du composant lipidique étant au maximum de 40 % en poids par rapport au poids total du composant liant.

2. Formulation suivant la revendication 1, **caractérisée en ce que** la formulation est essentiellement exempte de cristaux de substance active.

3. Formulation suivant la revendication 1 ou 2, **caractérisée en ce que** la substance active est difficilement soluble dans l'eau.

4. Formulation suivant la revendication 3, **caractérisée en ce que**, pour dissoudre 1 partie de la substance active difficilement soluble dans l'eau, au moins 100 parties d'eau sont nécessaires.

5. Formulation suivant l'une des revendications 1 à 4, **caractérisée en ce que** le lipide est choisi parmi des acides gras, des triglycérides, des diglycérides et des monoglycérides.

6. Formulation suivant la revendication 5, **caractérisée en ce que** le lipide est choisi parmi des acides gras insaturés ainsi que des monoglycérides, diglycérides ou triglycérides d'acides gras insaturés.

7. Formulation suivant la revendication 6, **caractérisée en ce que** l'acide gras insaturé est choisi parmi de l'acide oléique, de l'acide linoléique et de l'acide linolénique.

8. Formulation suivant l'une des revendications 1 à 7, **caractérisée en ce que** le composant lipidique présente une valeur HLB d'au maximum 12.

9. Formulation suivant l'une des revendications 1 à 8, **caractérisée en ce que** le composant lipidique présente un point de fusion inférieur à 30°C.

10. Formulation suivant l'une des revendications 1 à 9, **caractérisée en ce que** le composant lipidique se présente sous une forme dispersée à l'état moléculaire.

11. Formulation suivant l'une des revendications 1 à 10, **caractérisée en ce que** la fraction du composant lipidique est au maximum de 25 % en poids par rapport au composant liant.

12. Formulation suivant l'une des revendications 1 à 11, **caractérisée en ce que** le liant est choisi parmi des polyvinylpyrrolidones, des copolymères de vinylpyrrolidone-acétate de vinyle, des hydroxypropylcelluloses et des hydroxypropylméthylcelluloses.

13. Formulation suivant l'une des revendications 1 à 12, **caractérisée en ce que** l'autre adjuvant est choisi parmi des charges, des lubrifiants, des agents antifriction, des agents séparateurs, des agents de développement, des colorants, des stabilisants, des agents plastifiants, des agents réticulants, des conservateurs, des agents de libération, des agents adsorbants, des agents de démoulage et des agents tensioactifs.

14. Formulation suivant l'une des revendications 1 à 13, **caractérisée en ce qu'**elle peut être obtenue par extrusion à l'état fondu d'un mélange plastique contenant les composants i), ii), iii) et éventuellement iv).

15. Formulation autoémulsifiante, contenant
i') un composant de substance active formé d'au moins une huile essentielle,
ii') éventuellement une autre fraction de composant lipidique formée d'au moins un autre lipide,
iii) un composant liant comportant au moins un liant choisi parmi des polyvinylpyrrolidones, des copolymères de vinylpyrrolidone-acétate de vinyle, des hydroxyalkylcelluloses, des hydroxyalkyl-alkylcelluloses, des phtalates de cellulose, des résines (méth)acryliques, et
iv) éventuellement d'autres adjuvants,
la fraction des composants i') et ii') étant de 6 à 60 % en poids par rapport au poids total de la formulation et la fraction des composants i') et ii') étant d'au maximum 40 % en poids par rapport au composant liant.

16. Formulation suivant la revendication 15, **caractérisée en ce que** la fraction des composants i') et ii') est de 11 à 40 % en poids par rapport au poids total de la formulation.

17. Formulation suivant la revendication 15 ou 16, **caractérisée en ce que** la fraction d'huile essentielle est de 5 à 20 % en poids par rapport au poids total de la formulation.

18. Formulation suivant l'une des revendications 15 à 17, **caractérisée en ce que** le composant liant comporte aussi au moins un alcool de sucre et/ou au moins un produit de saccharification d'amidon.

19. Formulation suivant la revendication 18, **caractérisée en ce que** l'alcool de sucre est choisi parmi de la maltite, de la xylite et de l'isomalt.

20. Formulation suivant la revendication 18, **caractérisée en ce que** le produit de saccharification d'amidon est de la maltodextrine.

21. Formulation suivant l'une des revendications 1 à 20, **caractérisée en ce qu'**elle est solide.

22. Utilisation d'une formulation suivant l'une des revendications 15 à 21 dans des produits d'hygiène.
